# EUROPEAN PATENT APPLICATION

(11) **EP 3 981 789 A1**
(43) Date of publication of application: **13.04.2022**
(21) Application number: 20306197.3
(22) Date of filing: 12.10.2020
(51) Int. Cl.: C07K 16/28

(54) **ANTI-LILRB ANTIBODIES AND USES THEREOF**

(71) Applicant: COMMISSARIAT À L'ÉNERGIE ATOMIQUE ET AUX ÉNERGIES ALTERNATIVES, 75015 Paris (FR)
(72) Inventor: Favier, Benoit, 92260 Fontenay-aux-Roses (FR); Coindre, Sixtine, 92260 Fontenay-aux-Roses (FR); Wijkhuisen, Anne, 91191 Gif sur Yvette (FR); Simon, Stéphanie, 91191 Gif sur Yvette (FR); Legrand, Roger, 92260 Fontenay-aux-Roses (FR); Lambotte, Olivier, 94276 Le Kremlin Bicêtre (FR)
(74) Representative: Nony

(57) **Abstract**

The present disclosure relates to an antagonist antibody, or an antigen binding fragment thereof, that binds both to human and macaque LILRB1 and/or LILRB2. This disclosure also relates to diagnostic and therapeutic uses of these antibodies

SEQUENCE LISTING

<110> COMISSARIAT A L'ENERGIE ATOMIQUE ET AUX ENERGIES ALTERNATIVES

<120> Anti-LILRB antibodies and uses thereof

<130> PR87810

<160> 21

<170> BiSSAP 1.3.6

<210> 1
<211> 8
<212> PRT
<213> Artificial Sequence

<220>
<223> B1.2.1. - H-CDR1

<400> 1
Gly Phe Thr Phe Asn Thr His Ala
1               5

<210> 2
<211> 10
<212> PRT
<213> Artificial Sequence

<220>
<223> B1.2.1. - H-CDR2

<400> 2
Ile Arg Ser Lys Ser Ser Asn Tyr Ala Thr
1               5                   10

<210> 3
<211> 15
<212> PRT
<213> Artificial Sequence

<220>
<223> B1.2.1. - H-CDR3

<400> 3
Val Met Val Tyr Tyr Gly Asn Ser Pro Tyr Tyr Ser Met Asp Tyr
1               5                   10                  15

<210> 4
<211> 124
<212> PRT
<213> Artificial Sequence

<220>
<223> B1.2.1. - VH

<400> 4
Glu Val Gln Leu Val Glu Ser Gly Gly Gly Leu Val Gln Pro Lys Gly
1               5                   10                  15

Ser Leu Lys Leu Ser Cys Ala Ala Ser Gly Phe Thr Phe Asn Thr His
            20                  25                  30
Ala Met His Trp Val Arg Gln Ala Pro Gly Lys Gly Leu Glu Trp Val
        35                  40                  45
Ala Arg Ile Arg Ser Lys Ser Ser Asn Tyr Ala Thr Phe Tyr Ala Asp
    50                  55                  60
Ser Val Lys Asp Arg Phe Thr Ile Ser Arg Asp Asp Ser Gln Ser Met
65                  70                  75                  80
Leu Tyr Leu Gln Met Asn Asn Leu Lys Thr Glu Asp Thr Ala Ile Tyr
                85                  90                  95
Phe Cys Val Met Val Tyr Tyr Gly Asn Ser Pro Tyr Tyr Ser Met Asp
            100                 105                 110
Tyr Trp Gly Gln Gly Thr Ser Val Thr Val Ser Ser
        115                 120

<210> 5
<211> 6
<212> PRT
<213> Artificial Sequence

<220>
<223> B1.2.1. - L-CDR1

<400> 5
Glu Asn Val Gly Thr Tyr
1               5

<210> 6
<211> 3
<212> PRT
<213> Artificial Sequence

<220>
<223> B1.2.1. - L-CDR2

<400> 6
Gly Ala Ser
1

<210> 7
<211> 9
<212> PRT
<213> Artificial Sequence

<220>
<223> B1.2.1. - L-CDR3

<400> 7
Gly Gln Ser Tyr Ser Ser Pro Leu Thr
1               5

<210> 8
<211> 107
<212> PRT
<213> Artificial Sequence

<220>
<223> B1.2.1. - VL

<400> 8
Asn Ile Val Met Thr Gln Ser Pro Lys Ser Met Ser Met Ser Val Gly
1               5                   10                  15
Glu Arg Val Thr Leu Ser Cys Lys Ala Ser Glu Asn Val Gly Thr Tyr
            20                  25                  30
Val Ser Trp Tyr Gln Gln Lys Pro Glu Gln Ser Pro Lys Leu Leu Ile
        35                  40                  45
Tyr Gly Ala Ser Asn Arg Tyr Thr Gly Val Pro Asp Arg Phe Thr Gly
    50                  55                  60
Ser Gly Ser Ala Thr Asp Phe Thr Leu Ile Ile Asn Asn Ile Gln Ala
65                  70                  75                  80
Glu Asp Leu Ala Asp Tyr His Cys Gly Gln Ser Tyr Ser Ser Pro Leu
                85                  90                  95
Thr Phe Gly Ala Gly Thr Lys Leu Glu Leu Lys
            100                 105

<210> 9
<211> 8
<212> PRT
<213> Artificial Sequence

<220>
<223> B1.2.2. - H-CDR1

<400> 9
Gly Phe Thr Phe Ser Asp Tyr Gly
1               5

<210> 10
<211> 8
<212> PRT
<213> Artificial Sequence

<220>
<223> B1.2.2. - H-CDR2

<400> 10
Ile Ser Ser Gly Ser Ser Thr Ser
1               5

<210> 11
<211> 15
<212> PRT
<213> Artificial Sequence

<220>
<223> B1.2.2. - H-CDR3

<400> 11
Ala Arg Pro Gly Ser Asn Trp Asp Val Tyr Tyr Ala Met Asp Tyr
1               5                   10                  15

<210> 12
<211> 122
<212> PRT
<213> Artificial Sequence

<220>
<223> B1.2.2. - VH

<400> 12
Glu Val Gln Leu Val Glu Ser Gly Gly Gly Leu Val Lys Pro Gly Gly
1               5                   10                  15
Ser Leu Lys Leu Ser Cys Ala Ala Ser Gly Phe Thr Phe Ser Asp Tyr
            20                  25                  30
Gly Met His Trp Val Arg Gln Ala Pro Glu Lys Gly Leu Glu Trp Val
        35                  40                  45
Ala Tyr Ile Ser Ser Gly Ser Ser Thr Ser Tyr Tyr Ala Asp Thr Val
    50                  55                  60
Lys Gly Arg Phe Thr Ile Ser Arg Asp Asp Ala Lys Asn Thr Leu Phe
65                  70                  75                  80
Leu Gln Met Thr Ser Leu Arg Ser Glu Asp Thr Ala Met Tyr Tyr Cys
                85                  90                  95
Ala Arg Pro Gly Ser Asn Trp Asp Val Tyr Tyr Ala Met Asp Tyr Trp
            100                 105                 110
Gly Gln Gly Thr Ser Val Thr Val Ser Ser
        115                 120

<210> 13
<211> 10
<212> PRT
<213> Artificial Sequence

<220>
<223> B12.2. - L-CDR1

<400> 13
Gln Ser Val Ser Thr Ser Ser Tyr Ser Tyr
1               5                   10

<210> 14
<211> 3
<212> PRT
<213> Artificial Sequence

<220>
<223> B1.2.2. - L-CDR2

<400> 14
Asn Ala Ser
1

<210> 15
<211> 9
<212> PRT
<213> Artificial Sequence

<220>
<223> B1.2.2. - L-CDR-3

<400> 15
Gln His Ser Trp Glu Ile Pro Phe Thr
1               5

<210> 16
<211> 111
<212> PRT
<213> Artificial Sequence

<220>
<223> B1.2.2. - VL

<400> 16
Asp Ile Val Leu Thr Gln Ser Pro Ala Ser Leu Ala Val Ser Leu Gly
1               5                   10                  15
Gln Arg Ala Thr Ile Ser Cys Arg Ala Ser Gln Ser Val Ser Thr Ser
            20                  25                  30
Ser Tyr Ser Tyr Met His Trp Tyr Gln Gln Lys Pro Gly Gln Pro Pro
        35                  40                  45
Lys Leu Leu Ile Lys Asn Ala Ser Asn Leu Glu Ser Gly Val Pro Ala
    50                  55                  60
Arg Phe Ser Gly Ser Gly Ser Gly Thr Asp Phe Thr Leu Asn Ile His
65                  70                  75                  80
Pro Val Glu Glu Glu Asp Thr Ala Thr Tyr Tyr Cys Gln His Ser Trp
                85                  90                  95
Glu Ile Pro Phe Thr Phe Gly Gly Gly Thr Lys Leu Glu Ile Lys
            100                 105                 110

<210> 17
<211> 8
<212> PRT
<213> Artificial Sequence

<220>
<223> Flag sequence

<400> 17
Asp Tyr Lys Asp Asp Asp Asp Lys
1               5

<210> 18
<211> 15
<212> PRT
<213> Artificial Sequence

<220>
<223> Signal peptide

<400> 18
Met Ser Ala Leu Leu Ile Leu Ala Leu Val Gly Ala Ala Val Ala
1               5                   10                  15

<210> 19
<211> 10
<212> PRT
<213> Artificial Sequence

<220>
<223> MHC-I A*2402 HIV-1 Nef 134-143

<400> 19
Arg Tyr Pro Leu Thr Phe Gly Trp Cys Tyr
1               5                   10

<210> 20
<211> 9
<212> PRT
<213> Artificial Sequence

<220>
<223> MHC-I B*0801 HIV-1 Gag 259-267

<400> 20
Gly Glu Ile Tyr Lys Arg Trp Ile Ile
1               5

<210> 21
<211> 9
<212> PRT
<213> Artificial Sequence


<220>
<223> cynomolgus macaque Mafa-A1*063 SIV gag GW9

<400> 21
Gly Pro Arg Lys Pro Ile Lys Cys Trp
1               5

## Description

### FIELD OF THE DISCLOSURE

The present disclosure concerns the field of antagonizing the suppressor effects of LILRB1 and LILRB2 receptors, especially for therapeutic purpose.

### BACKGROUND OF THE DISCLOSURE

The leukocyte Ig-like receptor subfamily B (LILRB) is a group of type I transmembrane glycoproteins with extracellular Ig-like domains that bind ligands and intracellular ITIMs (for « Immunoreceptor Tyrosine-based Inhibitory Motif »).

Inhibitory leukocyte immunoglobulin-like receptors (LILRBs 1-5) transduce signals via intracellular immunoreceptor tyrosine-based inhibitory motifs (ITIMs) that recruit protein tyrosine phosphatase non-receptor type 6 (PTPN6 or SHP-1), protein tyrosine phosphatase non-receptor type 11 (PTPN11 or SHP-2), or Src homology 2 domain-containing inositol phosphatase (SHIP), leading to negative regulation of immune cell activation. The activation of certain LILRBs on immune cells by their ligands were shown to contribute to immune evasion by tumors. Recent studies found that several members of the LILRB subfamily are expressed by tumor cells, notably by hematopoietic cancer cells, and may directly regulate cancer development and relapse as well as the activity of cancer stem cells (Kang et al., 2016, Cell Cycle, Vol. 15 : 25-40).

Among LILRBs, LILRB 1 is the most widely distributed with expression on certain NK cells, monocytes/macrophages, eosinophils, basophils, dendritic cells, subsets of T cells, B cells, decidual macrophages, progenitor mast cells and osteoclasts. LILRB2 is expressed on, monocytes/macrophages, dendritic cells, basophils, decidual macrophages, mast cell progenitors, hematopoietic stem cells and osteoclasts.

Certain LILRBs have been shown to bind ligands. Among LILRBs, LILRB 1 and LILRB2 bind classical and non-classical MHC Class I molecules, which ligands activate the suppressor activity of these two receptors. Upon stimulation by ligands such as HLA-G on tumor cells, LILRB 1 and LILRB2 inhibit immune activation, thus indirectly supporting tumor development (Chen et al., 2018, J Clin Invest, Vol. 128 (12) : 5647-5662).

The dual roles as immune checkpoint molecules and tumor-sustaining factors has suggested that LILRBs, including LILRB1 and LILRB2, are relevant as targets for treating several diseases, which encompasses treating cancers. Noticeably, LILRBs, and especially LILRB2 are also involved in the immune response to viral infections, such as to HIV infection where up-regulation of LILRB2 and its MHC-I ligands on dendritic cells may account for immune dysregulation at a critical stage of the anti-viral response (Alaoui et al., 2018, Cellular and Molecular Life Sciences, Vol. 75 : 1871 - 1887). It has also been shown that an enhanced binding of viral peptide-MHC Class I complexes to LILRB2 on peripheral blood monocytes and dendritic cells may have a critical impact on their functional profile, specifically with regard to their ability for maturation and co-stimulatory molecule up-regulation (Lichterfeld et al., 2007, J Exp Med, Vol. 204 (12) : 2813-2824). LILRBs, and especially LILRB2, by regulating neutrophil functions, also have implications in inflammatory disorders, which encompasses those occurring during infection, including in case of sepsis (Baudhuin et al., 2013, Proc Natl Acad Sci USA, Vol. 110 (4) : 17957-17962).

Possible therapeutic approaches include using recombinant extracellular domains of these receptors or using blocking antibodies to competitively block their activity. It has been shown in the art that blocking both the MHC class I-LILRB1 signalling axis and CD47-SIRP-alpha signaling axis might sensitize tumors to macrophage attack and might indirectly enhance the function of other immune cells (Barkal et al., 2017, Nature Immunology, Vol. 19 (1) : 76-84). These authors believed that agents directed against the MHC class I-LILRB1 signaling axis might be an important component of therapeutic regimens that aim to engage the key effector functions of macrophages. It has also been shown that blocking LILRB1 (also termed "ILT2") and LILRB2 (also termed "ILT4") on certain dendritic cells enhanced the generation of cytotoxic T lymphocytes, which effect may have implication in case of viral infection and cancer (Banchereau et al., 2012, Proc Natl Acad Sci USA, Vol. 109 (46) : 18885 - 18890).

A plurality of antibodies directed to several members of LILRB subfamily have been described in the art. WO 2016/144728 discloses antibodies directed against human LILRB2, LILRB3 and LILRB4. This document describes the use of anti-human LILRB4 antibodies for the purpose of treating Acute Myeloid Leukemia (AML). WO 2019/144052 discloses antibodies binding to one or more of LILRB1, LILRB2, LILRB3, LILRB4 and LILRB5. These antibodies are prescribed for treating proliferative diseases, infectious diseases, neurological diseases or disorders as well as autoimmune diseases. WO 2020/061059 discloses anti-LILRB2 antibodies and their use for treating tumors.

There is still a need in the art for agents capable of blocking the suppressor activity of members of the LILRB subfamily, especially for therapeutic purpose, including for treating subjects affected with cancer, or with viral or bacterial infections, including those affected with sepsis.

### SUMMARY OF THE DISCLOSURE

The present disclosure relates to an antagonist antibody, or an antigen binding fragment thereof, that binds both to human and macaque LILRB 1 and/or LILRB2, wherein the said antagonist antibody is selected from the group consisting of:
A) an antagonist antibody AB1, or an antigen binding fragment thereof, comprising :
   (a) a heavy chain wherein the variable domain comprises :
      - a H-CDR1 having a sequence set forth as SEQ ID NO. 1 described therein,
      - a H-CDR2 having a sequence set forth as SEQ ID NO. 2 described therein, and
      - a H-CDR3 having a sequence set forth as SEQ ID NO. 3 described therein, and/or
   (b) a light chain wherein the variable domain comprises
      - a L-CDR1 having a sequence set forth as SEQ ID NO. 5 described therein,
      - a L-CDR2 having a sequence set forth as SEQ ID NO. 6 described therein, and
      - a L-CDR3 having a sequence set forth as SEQ ID NO. 7 described therein,
      and
B) an antagonist antibody AB2, or an antigen binding fragment thereof, comprising :
   (a) a heavy chain wherein the variable domain comprises :
      - a H-CDR1 having a sequence set forth as SEQ ID NO. 9 described therein,
      - a H-CDR2 having a sequence set forth as SEQ ID NO. 10 described therein, and
      - a H-CDR3 having a sequence set forth as SEQ ID NO. 11 described therein, and/or
   (b) a light chain wherein the variable domain comprises :
      - a L-CDR1 having a sequence set forth as SEQ ID NO. 13 described therein,
      - a L-CDR2 having a sequence set forth as SEQ ID NO. 14 described therein, and
      - a L-CDR3 having a sequence set forth as SEQ ID NO. 15 described therein.

In some embodiments, the antagonist antibody "AB1", or antigen binding fragment thereof, according to claim 1, comprising a heavy chain variable domain having at least 85% amino acid identity with SEQ ID NO. 4.

In some embodiments, the antagonist antibody "AB1", or antigen binding fragment thereof, according to claim 1, comprising a light chain variable domain having at least 85% amino acid identity with SEQ ID NO. 8.

In some embodiments, the antagonist antibody "AB2", or antigen binding domain fragment thereof, according to claim 1, comprising a heavy chain variable domain having at least 85% amino acid identity with SEQ ID NO. 12.

In some embodiments, the antagonist antibody "AB2", or antigen binding fragment thereof, according to claim 1, comprising a light chain variable domain having at least 85% amino acid identity with SEQ ID NO. 16.

In some embodiments, the antagonist antibody "AB1" or the antagonist antibody "AB2" is a chimeric antibody or a humanized antibody.

In some embodiments, the antagonist antibody "AB1" or the antagonist antibody "AB2", or antigen binding fragment thereof, is selected from the group consisting of Fv, Fab, F'ab')2, dsFv, scFv, sc(Fv)2 and diabodies.

This disclosure also relates to a nucleic acid comprising a sequence encoding an antagonist antibody, or antigen binding fragment thereof, as described herein, to a vector comprising such a nucleic acid, as well as to a host cell comprising such a nucleic acid or comprising such a vector.

This disclosure further pertains to a pharmaceutical composition comprising an antagonist antibody as disclosed herein and a pharmaceutically acceptable carrier.

This disclosure also concerns the antagonist antibody as described herein, for use as a medicament.

This disclosure also relates to the antagonist antibody as described herein, for its use for treating a disease or disorder selected in the group consisting of proliferative diseases, bacterial or viral infections including sepsis.

### DESCRIPTION OF THE FIGURES

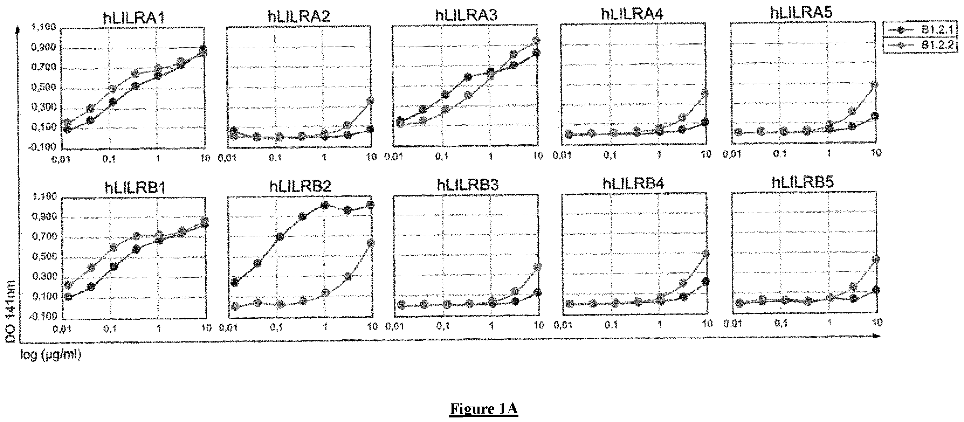

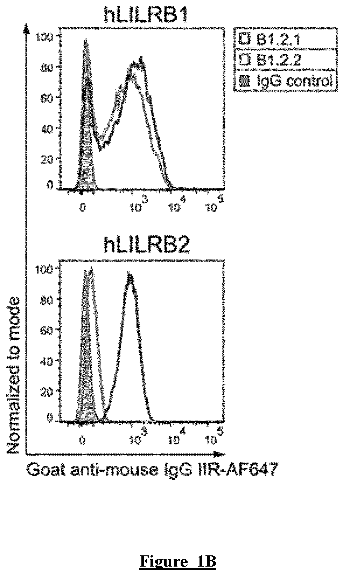

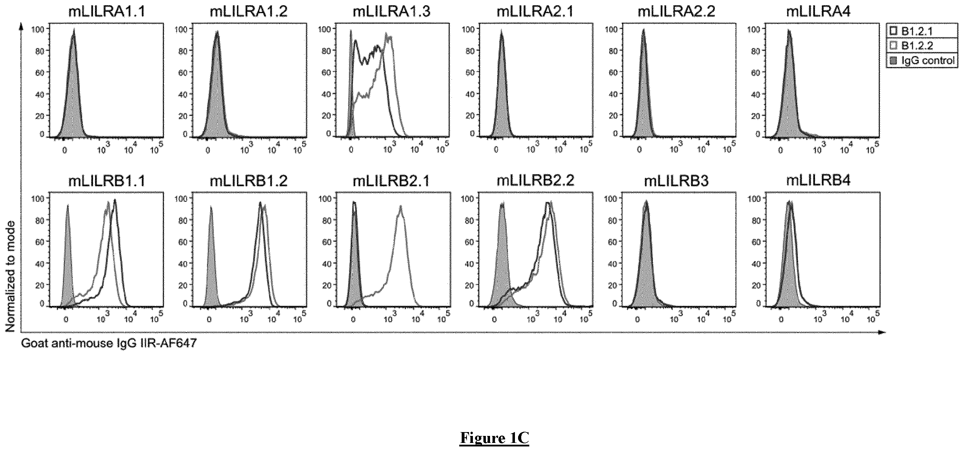

**Figure 1** **: Recognition of LILR from human and cynomolgus macaque origins by B1.2.1 and B1.2.2 monoclonal antibodies. (****Figure 1A****)** Recombinant human LILR-Fc fusion proteins recognition by B1.2.1 and B1.2.2 monoclonal antibodies using enzyme immunoassays (EIA). Recombinant human LILR-Fc fusion proteins were indirectly immobilized on the assay plate. Concentrations of B1.2.1 and B1.2.2 antibodies ranging from 0.01 ug/ml to 10 ug/ml were tested. Acetylcholinesterase (AChE)-labeled goat anti-mouse secondary antibody was used to detect receptors recognition by B1.2.1 and B1.2.2 antibodies. Dark line : Antibody B1.2.1. Grey line : B1.2.2. (**Figure 1B**) Recognition of human LILRB1 and LILRB2, expressed on stably transduced CHO cells, by B1.2.1 and B1.2.2 monoclonal antibodies using flow cytometry analysis. Dark line : Antibody B1.2.1. Grey line : B1.2.2. Grey area : IgG control(**Figure 1C**) Recognition of cynomolgus macaque LILRs, expressed by stably transduced HEK293T cells, by B1.2.1 and B1.2.2 monoclonal antibodies using flow cytometry analysis. Binding of monoclonal antibodies to human or macaque LILRs expressed by cell lines was detected using staining with a fluorescent-labeled goat anti-mouse IgG secondary antibody. Gray shaded histograms represent stainings with mouse IgG isotype control. Dark and Grey lines represent B1.2.1 and B1.2.2 monoclonal antibodies binding to LILRs, respectively. Dark line : Antibody B1.2.1. Grey line : B1.2.2. Grey area : IgG control.

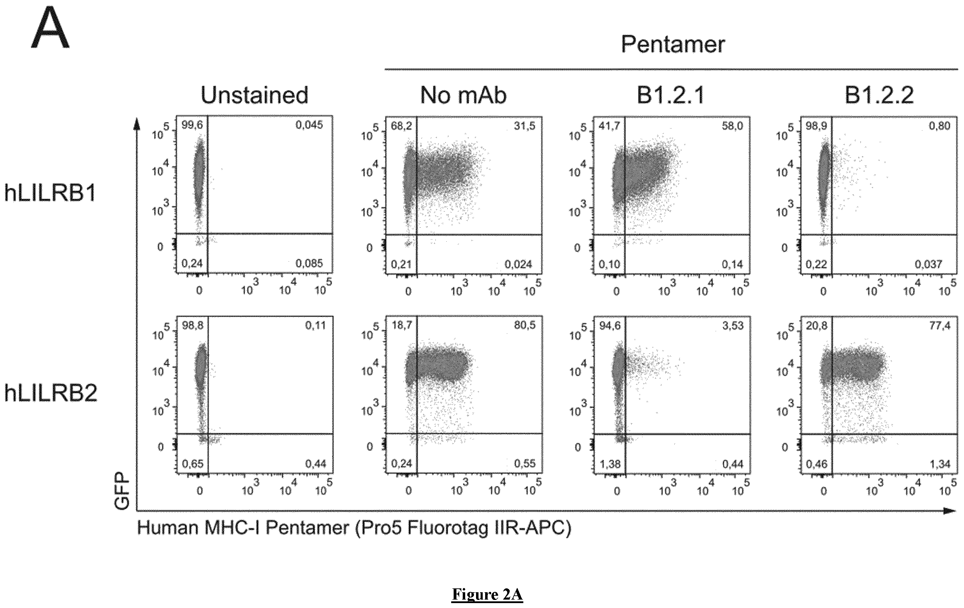

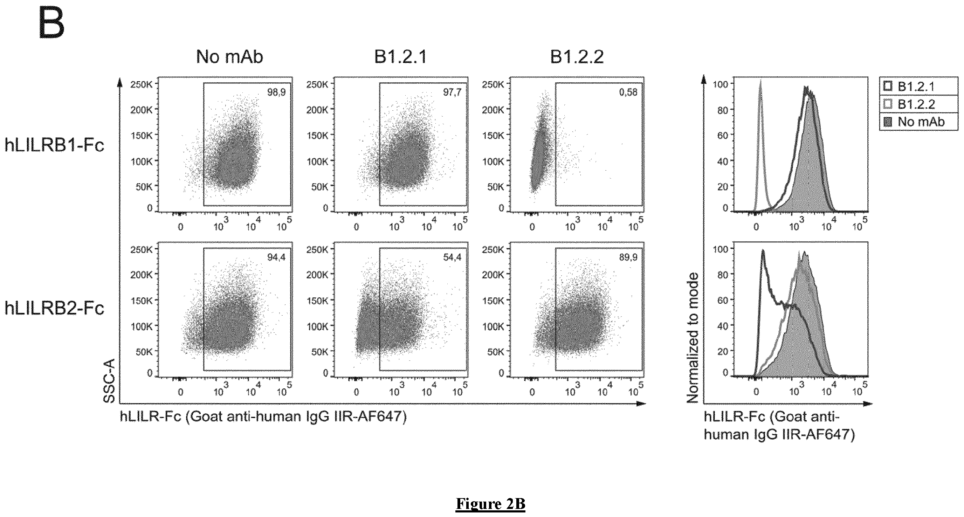

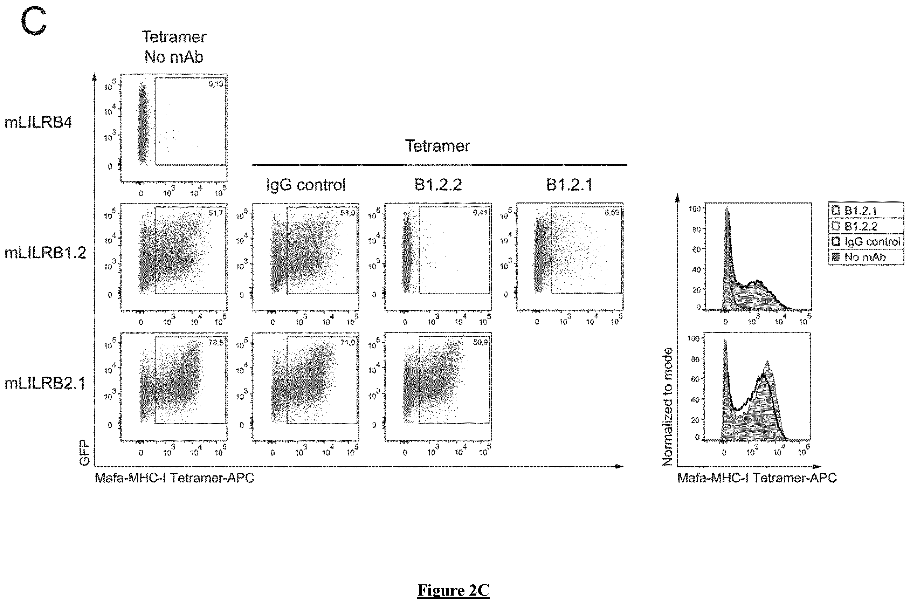

**Figure 2****: B1.2.1 and B1.2.2 monoclonal antibodies block LILRB1 and LILRB2 interactions with classical and non-classical MHC-I ligands from human and cynomolgus macaque.** B1.2.1 and B1.2.2 antibodies were co-incubated with cell lines expressing either human or macaque LILRB1 or LILRB2 or co-incubated with recombinant human LILRB1-Fc or LILRB2-Fc fusion protein. Their capacity to block the interaction between human or macaque LILRs with their MHC-I ligands was evaluated by flow cytometry analysis. (**Figure 2A**) Inhibition of human LILRB1 and LILRB2 (expressed on CHO cells) interaction with human MHC-I A*2402 HIV-1 Nef 134-143 (RYPLTFGWCY - SEQ ID NO. 19) pentamer (upper panel) and human MHC-I B*0801 HIV-1 Gag 259-267 (GEIYKRWII - SEQ ID NO. 20) pentamer (lower panel). (**Figure 2B**) Inhibition of human LILRB 1-Fc or LILRB2-Fc interaction with HLA-G1 expressed by M8 melanoma cells. Blocking capacity of B1.2.1 and B1.2.2 mAbs was evaluated using Alexa Fluor 647-labeled goat anti-human IgG secondary antibody. In the right panel, Dark line : Antibody B1.2.1. Grey line : B1.2.2. Grey peak : No monoclonal antibody. (**Figure 2C**) Inhibition of cynomolgus macaque LILRB 1.2 and LILRB2.1 (expressed on HEK293T cells) interactions with cynomolgus macaque APC labeled-Mafa-A1*063 SIV gag GW9 (GPRKPIKCW - SEQ ID NO. 21) tetramers. HEK293T cells expressing cynomolgus macaque LILRB4 were used as negative control to demonstrate the specificity of the Mafa-A1*063 SIV gag GW9 tetramer interaction. The gray shaded histograms represent LILRB1 or LILRB2 binding to Mafa-A1*063 SIV gag GW9 tetramer. Dark line and grey line represent binding of mLILRB1 or mLILRB2 to Mafa-A1*063 SIV gag GW9 after pre-incubation with B1.2.1 or B1.2.2 antibody, respectively. Black lines represent binding of mLILRB1 or mLILRB2 to Mafa-A1*063 SIV gag GW9 after pre-incubation with a mouse IgG isotype control. Grey area : no monoclonal antibody.

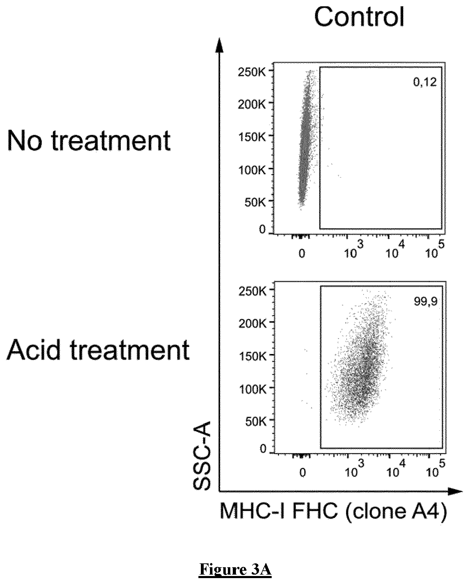

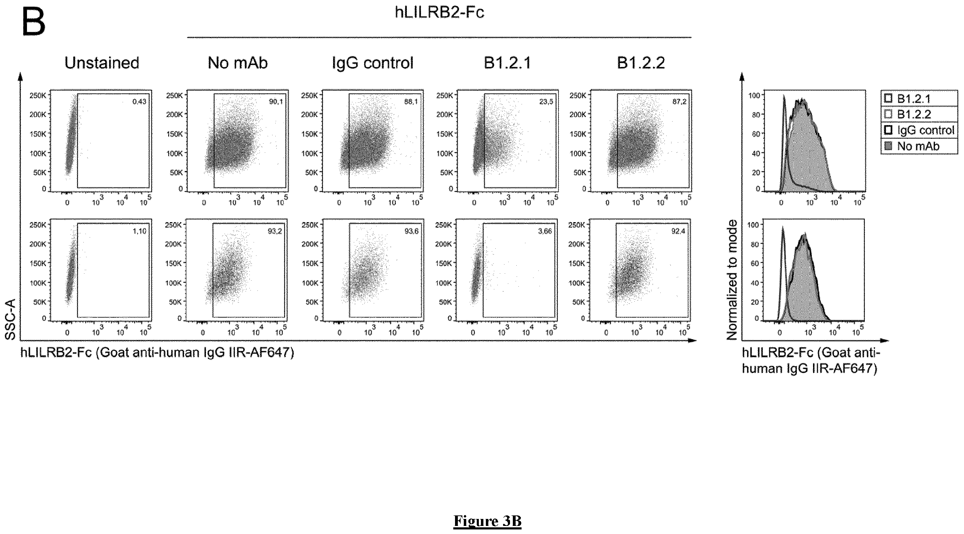

**Figure 3** **: B1.2.1 monoclonal antibody is able to block the interaction of human LILRB2 with non-classical HLA-I free heavy chain.** HLA-G1 stably transduced M8 cells were incubated in PBS1X (upper panel) or in acid solution (pH=3.5) (lower panel) to obtain MHC-I free heavy chains (FHC). The data depicted is representative of one out of 3 independent experiments. (**Figure 3A**) The complete dissociation of the beta-2-microglobulin from the HLA-G1 heavy chain was verified by staining the cells with a monoclonal antibody (clone A4) recognizing only MHC-I free heavy chain on the cells. (**Figure 3B**) B1.2.1 and B1.2.2 monoclonal antibodies were co-incubated with recombinant human LILRB2-Fc fusion protein. Their capacity to block the interaction of LILRB1 or -B2-Fc fusion protein with native HLA-G1 or HLA-G1 FHC was evaluated by flow cytometry using a goat anti-human IgG antibody labeled with Alexa Fluor 647. Data are representative of three independent experiments. Gray shaded histograms represent LILRB2-Fc fusion protein binding to M8-HLA-G1 cells (upper panel) or M8-HLA-G1 FHC cells (lower panel). Dark line and grey line represent LILRB2-Fc fusion protein binding after pre-incubation with B1.2.1 or B1.2.2 monoclonal antibody, respectively. Black lines represent LILRB2-Fc binding after pre-incubation with a mouse IgG isotype control. Grey area : No monoclonal antibody.

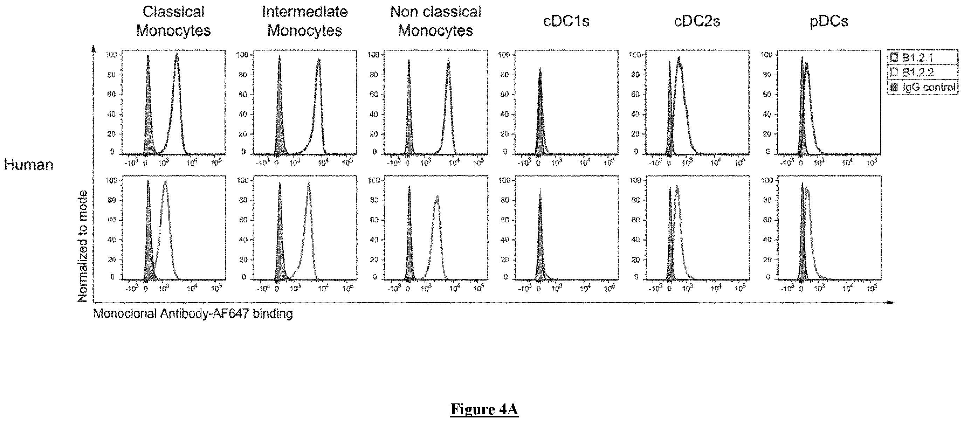

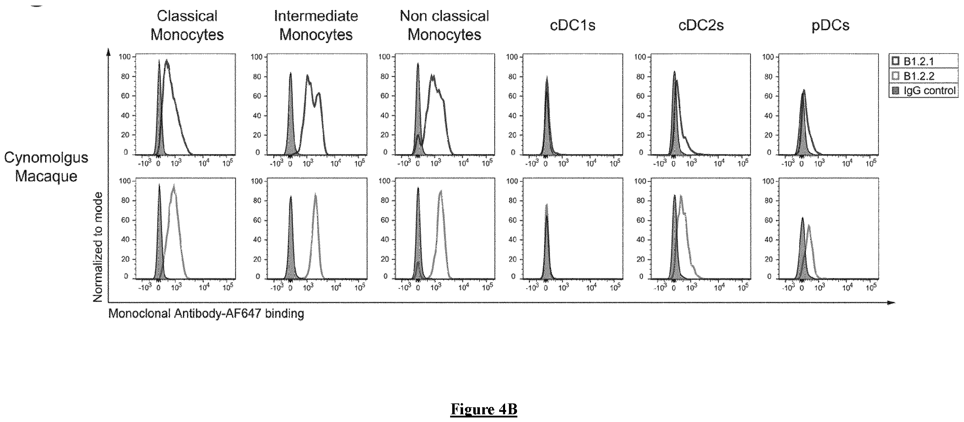

**Figure 4** **: Staining of human and cynomolgus macaque myeloid cell subsets with B1.2.1 and B1.2.2 monoclonal antibodies.** Flow cytometry analysis was performed on human and macaque PBMCs. Subsets of monocytes (classical, intermediate and non-classical monocytes) and of dendritic cells (cDC1s, cDC2s and pDCs) were characterized by specific surface markers. Recognition of each cell subset by B1.2.1 and B1.2.2 Alexa Fluor 647 conjugated antibodies was assessed. The data depicted is representative of 3 human and 3 macaque individuals. Myeloid subset gating strategy used on human and macaque PBMCs. The data depicted was generated from a macaque sample. (**Figure 4A**) Staining of human monocyte and dendritic cell subsets with Alexa Fluor 647-B1.2.1 or B1.2.2 monoclonal antibodies. Dark line : Antibody B1.2.1. Grey line : B1.2.2. Grey area : IgG control. (**Figure 4B**) Alexa Fluor 647-B1.2.1 or B1.2.2 mAbs distribution on cynomolgus macaque monocyte and dendritic cell subsets. Gray shaded histograms represent the mouse IgG isotype control binding to the cells. Dark red and light red lines represent B1.2.1 and B1.2.2 mAb binding to the cells, respectively. Dark line : Antibody B1.2.1. Grey line : B1.2.2. Grey area : IgG control.

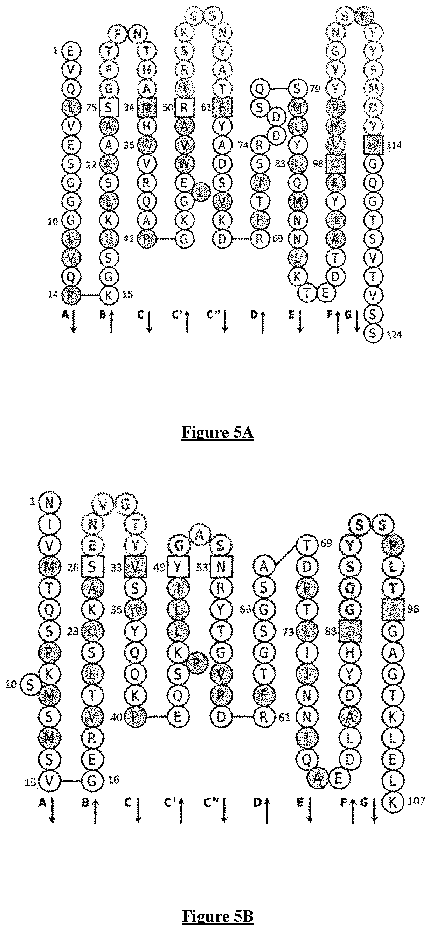

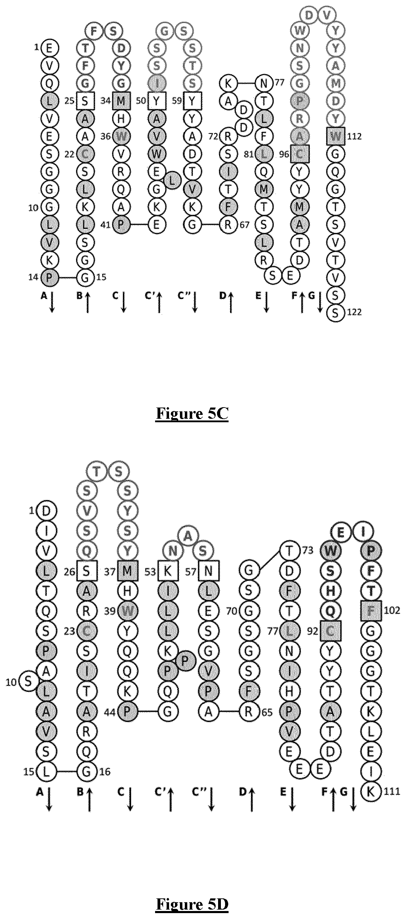

**Figure 5****: Graphical representation of variable domains of B1.2.1 and B1.2.2 monoclonal antibodies using IMGT labelling.** Figures 5A and 5B represent the amino acid sequence of B1.2.1 mouse monoclonal antibody (VH and VL, respectively) whereas figures 5C and 5D represents the amino acid sequence of B1.2.2 monoclonal antibody (VH and VL, respectively). For both antibodies, the consensus amino acid sequence from each variable domain was obtained from at least 5 different cDNA sequences then converted into amino acid sequences. Squares are key residues at the start and end of the CDRs..

### DETAILED DESCRIPTION OF THE DISCLOSURE

### Definitions

As used herein, "LILRB1" refers to the Leukocyte Immunoglobulin-like receptor subfamily B member 1. LILRB1 is a receptor for class I MHC antigens belonging to the LILRB subfamily. LILRB1 recognizes a broad spectrum of HLA-A, HLA-B, HLA-C, HLA-G and HLA-F alleles. Unless otherwise specified, LILRB 1 protein encompasses human LILRB 1 protein and macaque LILRB 1 protein herein. For the human LILRB 1 amino acid sequence, it may be referred to the UniProt database reference Q8NHL6 (LILRB 1 HUMAN). For the macaque LILRB 1 amino acid sequence, it may be referred to Slukvin et al. (2006, Tissue Antigens, Vol. 67(4): 331-337).

As used herein, "LILRB2" refers to Leukocyte Immunoglobulin-like receptor subfamily B member 2. LILRB2 is a receptor for class I MHC antigens belonging to the LILRB subfamily. LILRB2 recognizes a broad spectrum of HLA-A, HLA-B, HLA-C, HLA-G and HLA-F alleles. Unless otherwise specified, LILRB2 protein encompasses human LILRB2 protein and macaque LILRB2 protein herein. For the human LILRB2 amino acid sequence, it may be referred to the UniProt database reference Q8N423 (LILRB2_HUMAN. For the macaque LILRB2 amino acid sequence, it may be referred to the article of Slukvin et al. (2006, Tissue Antigens, Vol. 67(4) : 331-337).

According to the present disclosure, "antibody" or "immunoglobulin" have the same meaning and will be used equally in the present text. The term "antibody" as used herein refers to immunoglobulin molecules and encompasses immunologically active portions of immunoglobulin molecules, *i*.*e*., molecules that contain an antigen binding site that specifically binds a target antigen. As such, the term antibody encompasses not only whole antibody molecules, but also variants (including derivatives) of antibodies. In antibodies as used in the most conventional meaning of this term, two heavy chains are linked to each other by disulfide bonds and each heavy chain is linked to a light chain by a disulfide bond. There are two types of light chain, lambda (l) and kappa (k). There are five main heavy chain classes (or isotypes) which determine the functional activity of an antibody molecule: IgM, IgD, IgG, IgA and IgE. Each chain contains distinct sequence domains. The light chain includes two domains, a variable domain (VL) and a constant domain (CL). The heavy chain includes four domains, a variable domain (VH) and three constant domains (CH1, CH2 and CH3, collectively referred to as CH). The variable regions of both light (VL) and heavy (VH) chains determine binding recognition and specificity to the antigen. The constant region domains of the light (CL) and heavy (CH) chains confer important biological properties such as antibody chain association, secretion, trans-placental mobility, complement binding, and binding to Fc receptors (FcR). The Fv fragment is the N-terminal part of the Fab fragment of an immunoglobulin and consists of the variable portions of one light chain and one heavy chain. The specificity of the antibody resides in the structural complementarity between the antibody combining site and the antigenic determinant. Antibody combining sites are made up of residues that are primarily from the hypervariable or complementarity determining regions (CDRs). Occasionally, residues from non-hypervariable or framework regions (FR) influence the overall domain structure and hence the combining site. Complementarity Determining Regions or CDRs refer to amino acid sequences which together define the binding affinity and specificity of the natural Fv region of a native immunoglobulin binding site. The light and heavy chains of an immunoglobulin each have three CDRs, designated L-CDR1, L-CDR2, L-CDR3 and H-CDR1, H-CDR2, H-CDR3, respectively. An antigen-binding site of a natural antibody, therefore, includes six CDRs, comprising the CDR set from each of a heavy and a light chain V region. Framework Regions (FRs) refer to amino acid sequences interposed between CDRs.

Accordingly, in the present text, the term "antibody" is used in the broadest sense and includes fully assembled antibodies, monoclonal antibodies, polyclonal antibodies, multispecific antibodies (e.g., bispecific antibodies), antibody fragments and recombinant peptides comprising the foregoing as long as they exhibit the desired biological activity. Antibodies encompass heavy chain antibodies which consist only of two heavy chains and are lacking light chains.

The term "monoclonal antibody" as used herein refers to an antibody obtained from a population of substantially homogeneous antibodies, i.e., the individual antibodies comprising the population are identical except for possible naturally occurring mutations or alternative post-translational modifications that may be present in minor amounts. Monoclonal antibodies are highly specific; in contrast to conventional (polyclonal) antibody preparations that typically include different antibodies directed against different determinants (epitopes), each monoclonal antibody is directed against a single determinant on the antigen. In addition to their specificity, the monoclonal antibodies are advantageous in that they are synthesized by the homogeneous culture, uncontaminated by other immunoglobulins with different specificities and characteristics. For example, monoclonal antibodies in accordance with the disclosure may be made by the hybridoma method first described by Kohler et al., Nature 256:495 (1975), or may be made by recombinant DNA methods (see, e.g., U.S. Patent No. 4,816,567). The "monoclonal antibodies" may also be isolated from phage antibody libraries using the techniques described in Clackson et al., Nature 352:624-628 (1991) or Marks et al., J. Mol. Biol. 222:581-597 (1991), for example.

As used herein, an "antigen-binding fragment" of a given antibody that binds to a target antigen means an amino acid portion of the said given antibody (the said given antibody may be termed "parent antibody" or "starting antibody") that is endowed with the property of binding to the said target antigen. Taken into account the above definition of an "antibody", which definition encompasses molecules that contain an antigen binding site that specifically binds a target antigen, an antigen-binding fragment may be encompassed by the term "antibody" in its most general, but less conventional, meaning. Typically, an antigen binding fragment of a given antibody comprises at least three CDRs, such as at least the three CDRs originating from the variable domain of the light chain of the said given antibody or the three CDRs originating from the variable domain of the heavy chain of the said given antibody. An antigen binding fragment of an antibody encompasses in particular those selected from the group consisting of Fv, Fab, F(ab')2, Fab', dsFv, scFv, sc(Fv)2, single domain antibodies (sdAb, such as camel or llama antibodies), nanobodies (including single chain immunoglobulin termed "VHH") and diabodies. They may be produced by recombinant DNA techniques or by enzymatic or chemical cleavage of intact antibodies, according to methods well known to the man skilled in the art. As it is known in the art, a single domain antibody is an antibody fragment consisting of a single monomeric variable antibody domain. According to the present disclosure, an antigen binding fragment of a parent antibody has preferably an affinity for the target antigen which is of the same order of magnitude than the affinity of the parent antibody for the said target antigen, for example in affinity methods of measure involving a sensor bases on surface plasmon analysis, preferably according to BIAcore^{™} analysis. Due to their small size, these antibody fragments can be of high interest for example for generating small size immunoconjugates wherein the said antibody fragments are linked to a therapeutic agent.

"Single-chain Fv" or "scFv" antibody fragments comprise the VH and VL domains of antibody, wherein these domains are present in a single polypeptide chain. Generally, the Fv polypeptide further comprises a polypeptide linker between the VH and VL domains, which enables the scFv to form the desired structure for antigen binding. For a review of scFv, see Pluckthun in The Pharmacology of Monoclonal Antibodies, Vol 113, Rosenburg and Moore eds. Springer- Verlag, New York, pp. 269-315 (1994).

The term "diabodies" refers to small antibody fragments with two antigen-binding sites, which fragments comprise a heavy chain variable domain (VH) connected to a light chain variable domain (VL) in the same polypeptide chain (VH and VL). By using a linker that is too short to allow pairing between the two domains on the same chain, the domains are forced to pair with the complementary domains of another chain and create two antigen- binding sites. Diabodies are described more fully in, for example, EP 404,097; WO 93/11161; and Hollinger et al., Proc. Natl. Acad. Sci. USA, 90:6444-6448 (1993).

Diabodies or bi-specific antibodies can be roughly divided into two categories: immunoglobulin G (IgG)-like molecules and non-IgG-like molecules. IgG-like bsAbs retain Fc-mediated effector functions such as antibody-dependent cell-mediated cytotoxicity (ADCC), complement-dependent cytotoxicity (CDC), and antibody-dependent cellular phagocytosis (ADCP) (Spiess et al., 2015, Mol Immunol., Vol. 67(2) : 95-106.). The Fc region of bsAbs facilitates purification and improves solubility and stability. Bi-specific antibodies in IgG-like formats usually have longer serum half-lives owing to their larger size and FcRn-mediated recycling (Kontermann et al., 2015, Bispecific antibodies. Drug Discov Today Vol. 20(7) : 838-47). Non-IgG-like bsAbs are smaller in size, leading to enhanced tissue penetration (Kontermann et al., 2015, Bispecific antibodies. Drug Discov Today Vol. 20(7): 838-47).

A monoclonal antibody according to the present disclosure can in particular be a chimeric, humanized or human antibody.

The monoclonal antibodies specified herein specifically include "chimeric" anti-LILRB1 and anti-LILRB2 antagonist antibodies (immunoglobulins) in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (U.S. Patent No. 4,816,567; and Morrison et al., Proc. Natl. Acad. Sci. USA 81 :6851-6855 (1984)).

The monoclonal antibodies specified herein also encompass humanized anti-LILRB1 and anti-LILRB2 antagonist antibodies. "Humanized" forms of non-human (e.g., murine) antibodies are chimeric antibodies which contain minimal sequence derived from non-human immunoglobulin. For the most part, humanized antibodies are human immunoglobulins (recipient antibody) in which residues from a hypervariable region of the recipient are replaced by residues from a hypervariable region of a non-human species (donor antibody) such as mouse, rat, rabbit or nonhuman primate having the desired specificity, affinity, and capacity. In some instances, Fv framework region (FR) residues of the human immunoglobulin are replaced by corresponding non-human residues. Furthermore, humanized antibodies may comprise residues which are not found in the recipient antibody or in the donor antibody. These modifications are made to further refine antibody performance. In general, the humanized antibody will comprise substantially all of at least one, and typically two, variable domains, in which all or substantially all of the hypervariable loops correspond to those of a non-human immunoglobulin and all or substantially all of the FR regions are those of a human immunoglobulin sequence. The humanized antibody optionally also will comprise at least a portion of an immunoglobulin constant region (Fc), typically that of a human immunoglobulin. For further details, see Jones et al., Nature 321:522-525 (1986); Riechmann et al., Nature 332:323-329 (1988); and Presta, Curr. Op. Struct. Biol. 2:593-596 (1992). Humanized antibodies may be produced by obtaining nucleic acid sequences encoding CDR domains and constructing a humanized antibody according to techniques known in the art. Methods for producing humanized antibodies based on conventional recombinant DNA and gene transfection techniques are well known in the art (See, e.g., Riechmann L. *et al.* 1988; Neuberger M S. *et al.* 1985). Antibodies can be humanized using a variety of techniques known in the art including, for example, CDR-grafting (EP 239,400; PCT publication WO91/09967; U.S. Pat. Nos. 5,225,539; 5,530,101; and 5,585,089), veneering or resurfacing (EP 592,106; EP 519,596; Padlan E A (1991); Studnicka GM *et al.* (1994); Roguska M A. *et al.* (1994)), and chain shuffling (U.S. Pat. No. 5,565,332). The general recombinant DNA technology for preparation of such antibodies is also known (see European Patent Application EP 125023 and International Patent Application WO 96/02576).

As used herein, a "human antibody" is one which possesses an amino acid sequence which corresponds to that of an antibody produced by a human and/or has been made using any of the techniques for making human antibodies as disclosed herein. This definition of a human antibody specifically excludes a humanized antibody comprising non-human antigen-binding residues. Human antibodies can be produced using various techniques known in the art. In one embodiment, the human antibody is selected from a phage library, where that phage library expresses human antibodies (Vaughan et al. Nature Biotechnology 14:309-314 (1996): Sheets et al. Proc. Natl. Acad. Sci. 95:6157-6162 (1998)); Hoogenboom and Winter, J. Mol. Biol, 227:381 (1991); Marks et al., J. Mol. Biol, 222:581 (1991)). Human antibodies can also be made by introducing human immunoglobulin loci into transgenic animals, e.g., mice in which the endogenous immunoglobulin genes have been partially or completely inactivated. Upon challenge, human antibody production is observed, which closely resembles that seen in humans in all respects, including gene rearrangement, assembly, and antibody repertoire. This approach is described, for example, in U.S. Pat. Nos. 5,545,807; 5,545,806; 5,569,825; 5,625,126; 5,633,425; 5,661,016, and in the following scientific publications: Marks et al., Bio/Technology 10: 779-783 (1992); Lonberg et al., Nature 368: 856-859 (1994); Morrison, Nature 368:812-13 (1994); Fishwild et al., Nature Biotechnology 14: 845-51 (1996); Neuberger, Nature Biotechnology 14: 826 (1996); Lonberg and Huszar, Intern. Rev. Immunol. 13:65-93 (1995). Alternatively, the human antibody may be prepared via immortalization of human B lymphocytes producing an antibody directed against a target antigen (such B lymphocytes may be recovered from an individual or may have been immunized in vitro). See, e.g., Cole et al., Monoclonal Antibodies and Cancer Therapy, Alan R. Liss, p. 77 (1985); Boerner et al., J. Immunol, 147 (1):86-95 (1991); and U.S. Pat. No. 5,750,373.

Within the scope of the present disclosure, the "percentage identity" between two sequences of nucleic acids or peptides/proteins means the percentage of identical nucleotides or amino acid residues between the two sequences to be compared, obtained after optimal alignment, this percentage being purely statistical and the differences between the two sequences being distributed randomly along their length. The comparison of sequences is traditionally carried out by comparing the sequences after having optimally aligned them, said comparison being able to be conducted by segment or by using an "alignment window". Optimal alignment of the sequences for comparison can be carried out, in addition to comparison by hand, by means of the local homology algorithm of Smith and Waterman (1981), by means of the local homology algorithm of Neddleman and Wunsch (1970), by means of the similarity search method of Pearson and Lipman (1988) or by means of computer software using these algorithms (GAP, BESTFIT, FASTA and TFASTA in the Wisconsin Genetics Software Package, Genetics Computer Group, 575 Science Dr., Madison, WI, by the Geneious software (Biomatters, United States) or by the comparison software BLAST NR or BLAST P).

The term "subject" refers to any single subject for which diagnostic or therapy is desired or that is participating in a clinical trial, epidemiological study or used as a control, including humans, macaques and other mammalian veterinary patients. In certain preferred embodiments, the subject is a human.

The term "treatment" or "therapy" refers to administering an antibody, a cell or a composition according to the present disclosure with the purpose to cure, heal, alleviate, relieve, alter, remedy, ameliorate, improve, or affect a disease or a physiological state described herein, the symptoms of the condition, or to prevent or delay the onset of the symptoms, complications, biochemical indicia of a disease, or otherwise arrest or inhibit further development of the disease, condition, or disorder in a statistically significant manner.

By a "therapeutically effective amount" of an antagonist antibody according to the present disclosure, or of an antigen binding fragment thereof, is meant a sufficient amount of an antagonist antibody to treat the disease considered, at a reasonable benefit/risk ratio applicable to any medical treatment. The same applies, *mutatis mutandis*, to a composition and/or to a cell according to the disclosure. It will be understood, however, that the total daily usage of the antibodies, cells and compositions of the present disclosure will be decided by the attending physician within the scope of sound medical judgment. The specific therapeutically effective dose level for any particular patient will depend upon a variety of factors including the disorder being treated and the severity of the disorder; the activity of the specific antibody employed; the specific composition employed; the specific cell employed; the age, body weight, general health, sex and diet of the patient; the time of administration, route of administration, and rate of excretion of the specific antibody employed; the duration of the treatment; the drugs used in combination or coincidental with the specific antibody employed; and like factors well known in the medical arts. For example, it is well known within the skill of the art to start doses of the compound at levels lower than those required to achieve the desired therapeutic effect and to gradually increase the dosage until the desired effect is achieved.

The term "pharmaceutically acceptable" means what is useful in preparing a pharmaceutical composition generally safe, non-toxic, and neither biologically nor otherwise undesirable and includes what is acceptable for veterinary as well as human pharmaceutical use.

### Detailed disclosure

The present disclosure relates to antibodies directed to LILRB 1 and/or LILRB2 that may be used for therapeutic purpose.

There is provided herein antibodies directed to LILRB 1 and/or LILRB2 that recognize both human and non-human primates, including macaque (*e.g. Cynomolgus* macaque monkey) LILRB 1 and/or LILRB2. This dual property of binding both to human and macaque receptors allow the antibodies described herein to be used during the course of the successive steps of clinical trials, from the first clinical steps in a relevant animal model, here a primate animal model, to the clinical phases that are required in human subjects.

As it is known in the art, LILRB 1 sequences from non-human primates are close and are shared, notably by macaque monkeys (*e.g. Cynomolgus* macaque monkeys and *Rhesus* macaque monkeys), baboon, as well as African green monkeys, which non-human primates may thus be used for starting clinical trials of candidate therapeutic agents, including antibodies, that are aimed at binding to human LILRB 1. Exactly the same remarks also apply to LILRB2 sequences.

Two of these antibodies are termed "B.1.2.1" and "B.1.2.2" herein, respectively.

Accordingly, these antibodies bind both to the macaque and human targets LILRB 1 and/or LILRB2.

For the sake of clarity, macaque LILRB1 encompasses two isoforms which are termed "LILRB1.1" and "LILRB1.2.", respectively.

Similarly, macaque LILRB2 encompasses two isoforms which are termed "LILRB2.1" and "LILRB2.2", respectively.

As it is shown in the examples herein, B.1.2.1 antibody binds with both human and macaque LILRB1 as well as with human and macaque LILRB2, including when these receptors are expressed on cells.

As it is also shown in the examples herein, B.1.2.2 antibody binds with human and macaque LILRB1 and with macaque LILRB2, including when these receptors are expressed on cells.

Noticeably, none of the B.1.2.1 and B.1.2.2 antibodies binds with any of (i) the human LILRA2, LILRA4, LILRA5, LILRB3, LILRB4 nor LILRB5 nor with any of (ii) the macaque LILRA1.1, LILRA1.2, LILRA2.1, LILRA2.2, LILRA4, LILRB3 nor LILRB4.

Then, although the anti-LILRB1 and anti-LILRB2 antibodies described herein are endowed with cross-reactivity properties, when considering the human and macaque receptors, these antibodies show a substantial specificity for LILRB1 and/or LILRB2, which target specificity allows expecting reduced undesirable effects when administered to subjects in need thereof, or even an absence of undesirable effects when administered to subjects in need thereof.

As it will be specified further in the present disclosure, (i) the knowledge of the antigen binding site of the B.1.2.1 antibody has allowed conceiving a family of antibodies, and antigen binding fragments thereof, comprising the said antigen binding site, the said family of antibodies being termed family "AB1" in the present disclosure, and (ii) the knowledge of the antigen binding site of the B.1.2.2 antibody has allowed conceiving a family of antibodies, and antigen binding fragments thereof, comprising the said antigen binding site, the said family of antibodies being termed family "AB2" in the present disclosure. For the sake of clarity of the present disclosure, it is specified that (i) the family of antigen binding molecules antagonist of LILRB1 and/or LILRB2 which are derived from the starting B1.2.1 antibody may be termed "AB1" or "Family AB1" herein and (ii) the family of antigen binding molecules antagonist of LILRB1 and/or LILRB2 which are derived from the starting B1.2.1 antibody may be termed "AB2" or "Family AB2" herein.

Highly importantly, the B.1.2.1 and B.1.2.2 antibodies described herein are endowed with an antagonistic activity towards LILRB 1 and/or LILRB2, by blocking interaction of these receptors with their ligands, more precisely by blocking interaction of these receptors with their MHC Class I ligands, which consist either of classical or non-classical MHC Class I molecules. B1.2.1 antibody blocks interaction of human LILRB2 and macaque LILRB1 with classical and non-classical MHC Class I ligands. B1.2.2 antibody blocks interaction of both human and macaque LILRB1 and partially with macaque LILRB2 with classical and non-classical MHC Class I ligands.

Further importantly, the B1.2.1 antibody described herein also block the interaction of human LILRB2 with Class I MHC ligands devoid of the normally associated beta2-microglobulin, *i.e.* block the interaction between LILRB2 with Class I MHC free heavy chain. As shown in the examples herein, the B1.2.1 antibody blocks the interaction of human LILRB2 with non-classical HLA-I free heavy chain.

It is further shown in the examples that, in human and macaque, targets of B1.2.1 antibody are expressed in classical monocytes, intermediate monocytes, non-classical monocytes, as well as in certain dendritic cells such as conventional cDC2 (*i.e. CD45*+, *MHC-II*+, *CD11c*+, *CD1c*+) dendritic cells and plasmacytoid pDC (*i.e. CD45*+*, MHC-II*+, *CD123*+) dendritic cells.

It is also further shown in the examples that, in human and macaque, targets of B1.2.2 antibody are expressed in classical monocytes, intermediate monocytes, non-classical monocytes, as well as in certain dendritic cells such as conventional cDC2 (*i.e. CD45*+*,CD3⁻,CD20⁻,CD14⁻, MHC-II*+, *CD11c*+, *CD1c*+) dendritic cells and plasmacytoid pDC (*i.e. CD45*+*, CD3⁻, CD20⁻, CD14⁻ , MHC-II*+, *CD123*+) dendritic cells.

Altogether, the experimental data contained in the present disclosure support the provision of antagonist antibodies of human and macaque LILRB1 and/or LILRB2 which are useful, mainly, as therapeutic and diagnostic agents.

The present disclosure relates to an antagonist antibody, or an antigen binding fragment thereof, that binds both to human and macaque LILRB1 and/or LILRB2, wherein the said antagonist antibody is selected from the group consisting of:
A) an antagonist antibody, which may be termed "AB1" herein, or an antigen binding fragment thereof, comprising :
   (a) a heavy chain wherein the variable domain comprises :
      - a H-CDR1 having a sequence set forth as SEQ ID NO. 1,
      - a H-CDR2 having a sequence set forth as SEQ ID NO. 2, and
      - a H-CDR3 having a sequence set forth as SEQ ID NO. 3, and/or
   (b) a light chain wherein the variable domain comprises
      - a L-CDR1 having a sequence set forth as SEQ ID NO. 5,
      - a L-CDR2 having a sequence set forth as SEQ ID NO. 6, and
      - a L-CDR3 having a sequence set forth as SEQ ID NO. 7,
   and
B) an antagonist antibody, which may be termed "AB2." herein, or an antigen binding fragment thereof, comprising :
   (a) a heavy chain wherein the variable domain comprises :
      - a H-CDR1 having a sequence set forth as SEQ ID NO. 9,
      - a H-CDR2 having a sequence set forth as SEQ ID NO. 10, and
      - a H-CDR3 having a sequence set forth as SEQ ID NO. 11, and/or
   (b) a light chain wherein the variable domain comprises :
      - a L-CDR1 having a sequence set forth as SEQ ID NO. 13,
      - a L-CDR2 having a sequence set forth as SEQ ID NO. 14, and
      - a L-CDR3 having a sequence set forth as SEQ ID NO. 15,

Antibodies of the Family AB1 may be most preferably used as (i) antagonists of macaque LILRB1 and as (ii) antagonists of human LILRB2.

Antibodies of the Family AB2 may be most preferably used as (i) antagonists of human and macaque LILRB 1 and as (ii) antagonists of macaque LILRB2.

An "antagonist antibody" (or an antigen binding fragment thereof) refers to an antibody molecule (or an antigen binding fragment thereof) that is able to inhibit, and ideally block, the interaction of a human or macaque LILRB 1 or LILRB2 with one or more of its MHC Class I ligand.

In some embodiments, the said antagonistic antibody is designed so as to be endowed with a low immunogenicity with regard to the subject in need thereof, which allows performing a plurality of administrations to the said subject, possibly during a long period of time, while avoiding a strong immune response of the said subject against the said antagonistic antibody, which immune response may cause a reduced therapeutic efficacy.

By "low immunogenicity", it is meant that the said antagonistic antibody according to the present disclosure, in a particular embodiment, elicits no, or a reduced, humoral or cellular immune response, and preferably no, or reduced both humoral and cellular responses, in an animal having a functional immune system. Accordingly, an antagonistic antibody according to the disclosure can be unable to bind a Fc receptor. Such an antibody can be termed "Fcγ ablation variant" or "Fc knock out" (FcKO or KO) variant. Indeed, such variant is designed so as to remove the normal binding of the Fc domain, if present, to one or more or all of the Fcγ receptors (e.g. FcγR1, FcyRIIa, FcγRIIb, FcγRIIIa, etc.). Methods to obtain antibody variants are well known in the art, such as for example illustrated in Yamane-Ohnuki et al. (Biotechnol Bioeng 2004; 87(5): 614-622), Satoh et al. (Expert Opin Biol Ther 2006; 6(11): 1161-1173), Saunders (2019, Front. Immunol., Vol. 10, Article 1296), Shields et al. (2001, J Biol Chem, Vol. 276 : 6591-6604) and Dall'Acqua et al. (2002, J Immunol, Vol. 169 : S171-S180).

In some embodiments, an antagonist antibody according to the disclosure (Family AB1), or an antigen binding fragment thereof, comprises a heavy chain variable domain having at least 85% amino acid identity with SEQ ID NO. 4. Most preferably, according to these embodiments, the said antagonist antibody comprises the combination of CDRs of SEQ ID NO. 1, SEQ ID NO. 2 and SEQ ID NO. 3.

As used herein, a polypeptide having at least 85% amino acid identity with a reference polypeptide has at least 86%, at least 87%, at least 88%, at least 89%, at least 90%, at least 91%, at least 92%, at least 93%, at least 94%, at least 95%, at least 96%, at least 97%, at least 98% or at least 99% amino acid identity with the said reference polypeptide.

These embodiments encompass an antagonist antibody, or an antigen binding fragment thereof, comprising a heavy chain variable domain having 100% amino acid identity with SEQ ID NO. 4.

In some embodiments, an antagonist antibody according to the disclosure (Family AB1), or an antigen binding fragment thereof, comprises a light chain variable domain having at least 85% amino acid identity with SEQ ID NO. 8. Preferably, according to these embodiments, the said antagonist antibody comprises the combination of CDRs of SEQ ID NO. 5, SEQ ID NO. 6 and SEQ ID NO. 7.

These embodiments encompass an antagonist antibody, or an antigen binding fragment thereof, comprising a light chain variable domain having 100% amino acid identity with SEQ ID NO. 8.

In some embodiments, an antagonist antibody according to the disclosure (Family AB2), or an antigen binding fragment thereof, comprises a heavy chain variable domain having at least 85% amino acid identity with SEQ ID NO. 12. Preferably, according to these embodiments, the said antagonist antibody comprises the combination of CDRs of SEQ ID NO. 9, SEQ ID NO. 10 and SEQ ID NO. 11.

These embodiments encompass an antagonist antibody, or an antigen binding fragment thereof, comprising a heavy chain variable domain having 100% amino acid identity with SEQ ID NO. 12.

In some embodiments, an antagonist antibody according to the disclosure (Family AB2), or an antigen binding fragment thereof, comprises a light chain variable domain having at least 85% amino acid identity with SEQ ID NO. 16. Preferably, according to these embodiments, the said antagonist antibody comprises the combination of CDRs of SEQ ID NO. 13, SEQ ID NO. 14 and SEQ ID NO. 15.

These embodiments encompass an antagonist antibody, or an antigen binding fragment thereof, comprising a light chain variable domain having 100% amino acid identity with SEQ ID NO. 16.

This disclosure also relates to an antagonist antibody (Family AB1), or an antigen binding fragment thereof, that binds both to human and macaque LILRB 1 and/or LILRB2, wherein the said antagonist antibody comprises :
(a) a heavy chain wherein the variable domain comprises :
   - a H-CDR1 having a sequence set forth as SEQ ID NO. 1,
   - a H-CDR2 having a sequence set forth as SEQ ID NO. 2, and
   - a H-CDR3 having a sequence set forth as SEQ ID NO. 3, and
(b) a light chain variable domain having at least 85% amino acid identity with SEQ ID NO. 8.

Most preferably, the light chain variable domain (b) comprises (i) the L-CDR1 of SEQ ID NO. 5, (ii) the L-CDR2 of SEQ ID NO. 6 and (iii) the L-CDR3 of SEQ ID NO. 7.

In some embodiments, the light chain (b) has 100% amino acid identity with SEQ ID NO.8.

This disclosure also pertains to an antagonist antibody (Family AB1), or an antigen binding fragment thereof, that binds both to human and macaque LILRB 1 and/or LILRB2, wherein the said antagonist antibody comprises :
(a) a heavy chain variable domain having at least 85% amino acid identity with SEQ ID NO. 4, and
(b) a light chain wherein the variable domain comprises
   - a L-CDR1 having a sequence set forth as SEQ ID NO. 5,
   - a L-CDR2 having a sequence set forth as SEQ ID NO. 6, and
   - a L-CDR3 having a sequence set forth as SEQ ID NO. 7,

Most preferably, the heavy chain variable domain (a) comprises (i) the H-CDR1 of SEQ ID NO. 1, (ii) the H-CDR2 of SEQ ID NO. 2 and (iii) the H-CDR3 of SEQ ID NO. 3;

In some embodiments, the heavy chain (a) has 100% amino acid identity with SEQ ID NO.4.

This disclosure also concerns an antagonist antibody (Family AB2), or an antigen binding fragment thereof, that binds both to human and macaque LILRB 1 and/or LILRB2, wherein the said antagonist antibody comprises :
(a) a heavy chain wherein the variable domain comprises :
   - a H-CDR1 having a sequence set forth as SEQ ID NO. 9,
   - a H-CDR2 having a sequence set forth as SEQ ID NO. 10, and
   - a H-CDR3 having a sequence set forth as SEQ ID NO. 11, and
(b) a light chain variable domain having at least 85% amino acid identity with SEQ ID NO. 16.

Most preferably, the light chain variable domain (b) comprises (i) the L-CDR1 of SEQ ID NO. 13, (ii) the L-CDR2 of SEQ ID NO. 14 and (iii) the L-CDR3 of SEQ ID NO. 15.

In some embodiments, the light chain (b) has 100% amino acid identity with SEQ ID NO.16.

This disclosure also relates to an antagonist antibody (Family AB2), or an antigen binding fragment thereof, that binds both to human and macaque LILRB 1 and/or LILRB2, wherein the said antagonist antibody comprises :
(a) a heavy chain variable domain having at least 85% amino acid identity with SEQ ID NO. 12, and
(b) a light chain wherein the variable domain comprises :
   - a L-CDR1 having a sequence set forth as SEQ ID NO. 13,
   - a L-CDR2 having a sequence set forth as SEQ ID NO. 14 and
   - a L-CDR3 having a sequence set forth as SEQ ID NO. 15.

Most preferably, the heavy chain variable domain (a) comprises (i) the H-CDR1 of SEQ ID NO. 9, (ii) the H-CDR2 of SEQ ID NO. 10 and (iii) the H-CDR3 of SEQ ID NO. 11.

In some embodiments, the heavy chain (a) has 100% amino acid identity with SEQ ID NO.12.

This disclosure also pertains to an antagonist antibody (Family AB1), or an antigen binding fragment thereof, that binds both to human and macaque LILRB 1 and/or LILRB2, wherein the said antagonist antibody comprises :
(a) a heavy chain variable domain having at least 85% amino acid identity with SEQ ID NO. 4, and
(b) a light chain variable domain having at least 85% amino acid identity with SEQ ID NO. 8.

Most preferably, the heavy chain variable domain (a) comprises (i) the H-CDR1 of SEQ ID NO. 1, (ii) the H-CDR2 of SEQ ID NO. 2 and (iii) the H-CDR3 of SEQ ID NO. 3;

Most preferably, the light chain variable domain (b) comprises (i) the L-CDR1 of SEQ ID NO. 5, (ii) the L-CDR2 of SEQ ID NO. 6 and (iii) the L-CDR3 of SEQ ID NO. 7.

This disclosure also pertains to an antagonist antibody (Family AB2), or an antigen binding fragment thereof, that binds both to human and macaque LILRB 1 and/or LILRB2, wherein the said antagonist antibody comprises :
(a) a heavy chain variable domain having at least 85% amino acid identity with SEQ ID NO. 12, and
(b) a light chain variable domain having at least 85% amino acid identity with SEQ ID NO. 16.

Most preferably, the heavy chain variable domain (a) comprises (i) the H-CDR1 of SEQ ID NO. 9, (ii) the H-CDR2 of SEQ ID NO. 10 and (iii) the H-CDR3 of SEQ ID NO. 11.

Most preferably, the light chain variable domain (b) comprises (i) the L-CDR1 of SEQ ID NO. 13, (ii) the L-CDR2 of SEQ ID NO. 14 and (iii) the L-CDR3 of SEQ ID NO. 15.

In some embodiments, an antagonist antibody according to the present disclosure, or an antigen binding fragment thereof, is selected from the group consisting of Fv, Fab, F'ab')2, dsFv, scFv, sc(Fv)2 and diabodies.

In some embodiments, an antagonist antibody as described herein is a chimeric antibody comprising (i) constant regions of human origin and (ii) variable regions of non-human origin, such as variable regions of murine origin. In some embodiments of such a chimeric antibody, (il) the heavy chain constant region is of the human allotype G1m17.1 and bears the known LALA and YTE mutations and (i2) the light chains are of the human allotype Km3.

In some other embodiments, an antagonist antibody as described herein is a humanized antibody, that retains the variable region amino acid residues that are required for antigen binding. In some of these embodiments, the said humanized antibodies mostly comprise antibody sequences of human origin, excepted the complementary determining regions which are of non-human origin, e.g. which are of murine origin, and are grafted therein.

Designing the sequences of the VH and VL region of a human antibody comprising the CDRs of an antagonist antibody according to the present disclosure are notably disclosed by Couto et al. (1995, Cancer Research, Vol. 55 : 5973-5977)..

In some embodiments, an antagonist antibody according to the present disclosure comprises constant regions including a Fragment Crystallizable (Fc) domain derived from a macaque antibody or derived from a human antibody. Constant regions including Fc domains of macaque or human antibodies are well known in the art. In preferred embodiments, an antagonist antibody according to the present disclosure comprises constant regions of a human IgG antibody, wherein, in some embodiments, the said Fc domain comprises the YTE and the LALA mutations such as disclosed by Sauders (2019, Front. Immunol., Vol. 10, Article 1296). Designing an appropriate Fc domain of a human antibody, especially of a human IgG antibody is notably disclosed by Lobner et al. (1016, Immunological Reviews, Vol. 270 : 113-131).

In some embodiments, an antagonist antibody of family AB1, including antibody B1.2.1, or an antigen binding fragment thereof, as described herein, has a Kd affinity value for macaque LILRB1 of 10⁻¹⁰ M or less.

In some embodiments, an antagonist antibody of family AB1, including antibody B1.2.1, or an antigen binding fragment thereof, as described herein, has a Kd affinity value for macaque LILRB2 of 3 x 10⁻⁹ M or less.

In some embodiments, an antagonist antibody of family AB1, including antibody B1.2.1, or an antigen binding fragment thereof, as described herein, has a Kd affinity value for human LILRB 1 of 10⁻⁹ M or less.

In some embodiments, an antagonist antibody of family AB1, including antibody B1.2.1, or an antigen binding fragment thereof, as described herein, has a Kd affinity value for human LILRB2 of 2 x 10⁻¹⁰ M or less.

In some embodiments, an antagonist antibody of family AB2, including antibody B1.2.2, or an antigen binding fragment thereof, as described herein, has a Kd affinity value for macaque LILRB 1 of 4 x 10⁻⁹ M or less.

In some embodiments, an antagonist antibody of family AB2, including antibody B1.2.2, or an antigen binding fragment thereof, as described herein, has a Kd affinity value for macaque LILRB2 of 5 x 10⁻¹⁰ M or less.

In some embodiments, an antagonist antibody of family AB2, including antibody B1.2.2, or an antigen binding fragment thereof, as described herein, has a Kd affinity value for human LILRB 1 of 10⁻⁹ M or less.

In another aspect, provided herein is a nucleic acid comprising a sequence encoding any one of the antagonist antibodies, or antigen binding fragments thereof, that are described in the present disclosure.

In another aspect, provided herein is a vector comprising a nucleic acid encoding any one of the antagonist antibodies, or antigen binding fragments thereof, that are described in the present disclosure.

In another aspect, provided herein is a vector comprising a first nucleic acid and a second nucleic acid, wherein the first nucleic acid encodes the VH of any one of the antagonist antibodies or antigen binding fragments thereof, and wherein the second nucleic acid encodes the VL of the said antagonist antibody or antigen binding fragment thereof.

In another aspect, provided herein is a host cell comprising one or more nucleic acids encoding any one of the antagonist antibodies, or antigen binding fragments thereof or a vector comprising a nucleic acid encoding any one of the antagonist antibodies, or antigen binding fragments thereof that are described in the present disclosure. Host cells encompass prokaryotic cells and eukaryotic cells.

In another aspect, provided herein id a method for producing any one of the antagonist antibodies according to the present disclosure, or antigen binding fragments thereof, comprising the steps of:
a) culturing a host cell comprising one or more nucleic acids encoding any one of the antagonist antibodies according to the present disclosure, or antigen binding fragments thereof, under conditions suitable for expression of the said antibody or antigen binding fragment thereof, and
b) recovering the antibody or the antigen binding fragment thereof.

### Medical uses

As it is already disclosed elsewhere in the present specification, antagonist antibodies of Family AB1 and of Family AB2, and antigen-binding fragments thereof, consist of active ingredients that may be used for preventing or treating a number of diseases or disorders involving a state of immunosuppression. Thus, antagonist antibodies of Family AB1 and of Family AB2 according to the present disclosure, and the antigen-binding fragments thereof, consist of active ingredients that are useful for stimulating an immune response, or for restoring an effective immune response, in subjects in need thereof.

This disclosure also relates to an antagonist antibody, or an antigen binding fragment thereof, that binds both to human and macaque LILRB 1 and/or LILRB2, as described herein, for its use as a medicament.

This disclosure also pertains to the use of an antigen binding fragment thereof, that binds both to human and macaque LILRB1 and/or LILRB2, as described herein, for preparing a medicament.

As described throughout the present disclosure, antibodies of Family AB1 and of Family AB2, and the antigen-binding fragments thereof, consist of active ingredients for preventing or treating diseases or disorders involving an undesirable excess of expression of one of LILRB1 and LILRB2 receptors, or of both of them, or involving an undesirable level of activation of one of LILRB1 and LILRB2 receptors, or of both of them, which include proliferative diseases as well as viral or bacterial infections, including sepsis.

Thus, in some embodiments, this disclosure relates to an antagonist antibody, or an antigen binding fragment thereof, that binds both to human and macaque LILRB1 and/or LILRB2, as described herein, for its use for preventing or treating a subject affected with a disease or disorder involving a state of immunosuppression.

In some embodiments, this disclosure relates to an antagonist antibody, or an antigen binding fragment thereof, that binds both to human and macaque LILRB 1 and/or LILRB2, as described herein, for its use for preventing or treating a subject in need of stimulating an immune response or in need of blocking an immunosuppression state.

In some embodiments, this disclosure relates to an antagonist antibody, or an antigen binding fragment thereof, that binds both to human and macaque LILRB 1 and/or LILRB2, as described herein, for its use for preventing or treating a subject affected with a disease or disorder involving an undesirable excess of expression of one of LILRB1 and LILRB2 receptors, or of both of them, or involving an undesirable level of activation of one of LILRB 1 and LILRB2 receptors, or of both of them.

In some embodiments, this disclosure relates to an antagonist antibody, or an antigen binding fragment thereof, that binds both to human and macaque LILRB 1 and/or LILRB2, as described herein, for its use for preventing or treating a subject affected with a disease or disorder selected in a group comprising proliferative diseases, viral or bacterial infections, including sepsis.

This disclosure concerns the use of an antagonist antibody, or an antigen binding fragment thereof, that binds both to human and macaque LILRB 1 and/or LILRB2, as described herein, for the preparation of a medicament for preventing or treating a subject affected with a disease or disorder involving a state of immunosuppression.

This disclosure concerns the use of an antagonist antibody, or an antigen binding fragment thereof, that binds both to human and macaque LILRB 1 and/or LILRB2, as described herein, for the preparation of a medicament for preventing or treating a subject in need of stimulating an immune response or in need of blocking an immunosuppression state.

This disclosure concerns the use of an antagonist antibody, or an antigen binding fragment thereof, that binds both to human and macaque LILRB 1 and/or LILRB2, as described herein, for the preparation of a medicament for preventing or treating a subject affected with a disease or disorder involving an undesirable excess of expression of one of LILRB1 and LILRB2 receptors, or of both of them, or involving an undesirable level of activation of one of LILRB 1 and LILRB2 receptors, or of both of them.

This disclosure concerns the use of an antagonist antibody, or an antigen binding fragment thereof, that binds both to human and macaque LILRB 1 and/or LILRB2, as described herein, for the preparation of a medicament for preventing or treating a disease or disorder selected in a group comprising proliferative diseases, viral or bacterial infections, including sepsis.

This disclosure also pertains to a method for preventing or treating a subject affected with a disease or disorder involving a state of immunosuppression wherein the said method comprises a step of administering to the said subject an antagonist antibody, or an antigen binding fragment thereof, that binds both to human and macaque LILRB 1 and/or LILRB2, as described herein.

This disclosure also pertains to a method for preventing or treating a subject in need of stimulating an immune response or in need of blocking an immunosuppression state wherein the said method comprises a step of administering to the said subject an antagonist antibody, or an antigen binding fragment thereof, that binds both to human and macaque LILRB 1 and/or LILRB2, as described herein.

This disclosure also pertains to a method for preventing or treating a subject affected with a disease or disorder involving an undesirable excess of expression of one of LILRB 1 and LILRB2 receptors, or of both of them, or involving an undesirable level of activation of one of LILRB1 and LILRB2 receptors, or of both of them, wherein the said method comprises a step of administering to the subject an antagonist antibody, or an antigen binding fragment thereof, that binds both to human and macaque LILRB 1 and/or LILRB2, as described herein.

This disclosure also pertains to a method for preventing or treating a subject affected with a disease or disorder selected in a group comprising proliferative diseases, viral or bacterial infections, including sepsis, wherein the said method comprises a step of administering to the said subject an antagonist antibody, or an antigen binding fragment thereof, that binds both to human and macaque LILRB 1 and/or LILRB2, as described herein.

The antagonist antibodies according to the present disclosure, or the antigen binding fragments thereof, may be administered to a subject in need thereof, which includes a macaque and a human, by a variety of methods. For many applications, the route of administration is selected in a group comprising intravenous injection or parenteral administration, subcutaneous injection, intraperitoneal injection, intramuscular injection, local injection such as intra-tumoral injection.

In some embodiments, a subject may be assigned as being eligible to a treatment with an antagonist antibody, or an antigen binding fragment thereof, that binds both to human and macaque LILRB 1 and/or LILRB2, as described herein, by performing a method of detecting cell surface expression of LILRB1 and/or LILRB2 on cells of a tissue sample previously obtained from the said subject.

Detection of cell surface LILRB1 and/or LILRB2 protein expression may be performed according to a variety of methods that are well known from the one skilled in the art. Cell surface LILRB1 and/or LILRB2 protein expression detection methods notably encompass immunohistochemistry methods as well as fluorescence activated cell sorting methods.

This disclosure also relates to a method for determining whether a subject is eligible (*i.e.* responsive) to a treatment with an antagonist antibody, or an antigen binding fragment thereof, that binds both to human and macaque LILRB1 and/or LILRB2, as described herein, wherein the said method comprises the step of determining whether cells or a tissue sample previously obtained from the said subject expresses, or over-expresses, the LILRB 1 and/or LILRB2 protein at the cell surface.

Cells or tissue samples that may be used for determining whether a subject is eligible to a treatment with an antagonist antibody, or an antigen binding fragment thereof, that binds both to human and macaque LILRB 1 and/or LILRB2 encompass samples containing cells from immune system such as dendritic cells,, monocytes, macrophages, neutrophils, eosinophils, natural killer cells. Such cells or tissue samples encompass tissue samples derived from a tumor tissue microenvironment, which comprises cells of the immune systems that are susceptible, in certain cancer subjects, to be prevented from activation due to the binding of LILRB1 and/or LILRB2 to a MHC Class I ligand, including non-classical MHC-I molecules, associated or not to beta-2-microglobulin that is expressed by tumor cells or soluble MHC Class I ligand found in the tumor microenvironment.

In some embodiments, such a tissue sample consists of a whole blood sample, or alternatively of a whole blood-derived sample, which encompasses a PBMC (Peripheral Blood Mononuclear Cells) sample.

In some other embodiments, such a tissue sample consists of a tumor tissue sample previously obtained by performing a biopsy in a subject affected with a cancer.

Most preferably, the said tissue sample consists of a human tissue sample or of a macaque tissue sample.

This disclosure also relates to an *in vitro* method for determining whether a subject is eligible (*i.e.* responsive) to a treatment with an antagonist antibody, or an antigen binding fragment thereof, that binds both to human and macaque LILRB1 and/or LILRB2, as described herein, wherein the said method comprises the steps of :
a) determining if cells contained in a sample previously obtained from the said patient express, or over-express, LILRB 1 and/or LILRB2 at their membrane, and
b) concluding that the said subject is eligible to a treatment (*i.e.* is responsive to a treatment ) with an an antagonist antibody, or an antigen binding fragment thereof, that binds both to human and macaque LILRB1 and/or LILRB2, if membrane expression, or over-expression, of LILRB1 and/or LILRB2 by the said cells has been determined at step a).

Determining expression, or over-expression, of macaque LILRB1 may be performed with an antagonist antibody of Family AB1 disclosed herein, or an antigen binding fragment thereof.

Determining expression, or over-expression, of macaque LILRB 1 may be performed with an antagonist antibody of Family AB2 disclosed herein, or an antigen binding fragment thereof.

Determining expression, or over-expression, of macaque LILRB2 may be performed with an antagonist antibody of Family AB2 disclosed herein, or an antigen binding fragment thereof.

Determining expression, or over-expression, of macaque LILRB2 may be performed with an antagonist antibody of Family AB1 disclosed herein, or an antigen binding fragment thereof. Determining expression, or over-expression, of human LILRB1 may be performed with an antagonist antibody of Family AB1 disclosed herein, or an antigen binding fragment thereof.

Determining expression, or over-expression, of human LILRB2 may be performed with an antagonist antibody of Family AB1 disclosed herein, or an antigen binding fragment thereof.

Determining expression, or over-expression, of human LILRB1 may be performed with an antagonist antibody of Family AB2 disclosed herein, or an antigen binding fragment thereof.

Determining expression, or over-expression, of both macaque LILRB 1 and LILRB2 may be performed with an antagonist antibody of Family AB1 disclosed herein, or an antigen binding fragment thereof.

Determining expression, or over-expression, of both macaque LILRB 1 and LILRB2 may be performed with an antagonist antibody of Family AB2 disclosed herein, or an antigen binding fragment thereof.

Determining expression, or over-expression, of both human LILRB1 and LILRB2 may be performed with an antagonist antibody of Family AB1 disclosed herein, or an antigen binding fragment thereof.

Over-expression of LILRB1 or LILRB2 may be determined by comparing the LILRB1 or LILRB2 expression level which is determined at step a) of the method with a reference level of expression of the same receptor in healthy subjects, such as the mean level of expression of the same receptor in healthy subjects.

### Proliferative diseases

By "proliferative disease" is meant a disease which is caused by, or results in, inappropriately high levels of cell division, inappropriately lowlevels of apoptosis, or both. For example, cancers such as lymphoma, leukemia, melanoma, ovarian cancer, breast cancer, pancreatic cancer, and lung cancer are all examples of proliferative diseases.

A "cancer" refers to a broad group of various diseases characterized by the uncontrolled growth of abnormal cells in the body. Unregulated cell division and growth divide and grow results in the formation of malignant tumors that invade neighboring tissues and may also metastasize to distant parts of the body through the lymphatic system or bloodstream.

As used herein, cancers encompass liver cancer, bone cancer, pancreatic cancer, skin cancer, cancer of the head or neck, breast cancer, lung cancer, cutaneous or intraocular malignant melanoma, renal cancer, uterine cancer, ovarian cancer, colorectal cancer, colon cancer, rectal cancer, cancer of the anal region, stomach cancer, testicular cancer, uterine cancer, carcinoma of the fallopian tubes, carcinoma of the endometrium, carcinoma of the cervix, carcinoma of the vagina, carcinoma of the vulva, non-Hodgkin's lymphoma, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, solid tumors of childhood, lymphocytic lymphoma, cancer of the bladder, cancer of the kidney or ureter, carcinoma of the renal pelvis, neoplasm of the central nervous system (CNS), primary CNS lymphoma, tumor angiogenesis, spinal axis tumor, brain stem glioma, pituitary adenoma, Kaposi's sarcoma, epidermoid cancer, squamous cell cancer, environmentally induced cancers including those induced by asbestos, hematologic malignancies including, for example, multiple myeloma, B-cell lymphoma, Hodgkin lymphoma/primary mediastinal B-cell lymphoma, non-Hodgkin's lymphomas, acute myeloid lymphoma, chronic myelogenous leukemia, chronic lymphoid leukemia, follicular lymphoma, diffuse large B-cell lymphoma, Burkitt's lymphoma, immunoblastic large cell lymphoma, precursor B-lymphoblastic lymphoma, mantle cell lymphoma, acute lymphoblastic leukemia, mycosis fungoides, anaplastic large cell lymphoma, T-cell lymphoma, and precursor T-lymphoblastic lymphoma, and any combinations of said cancers. The present disclosure is also applicable to treatment of metastatic cancers.

### Viral infections

Viral infections may occur after immunosuppression due to reactivation of viruses already present in the recipient. Specific examples of persistent viral infections include cytomegalovirus (CMV) pneumonia, enteritis, and retinitis; Epstein-Barr virus (EBV) lymphoproliferative disorder; fowlpox / zoster virus (varicella-zoster virus, Caused by VZV); HSV-1 and -2 mucositis; HSV-6 encephalitis; BK-virus hemorrhagic cystitis; viral influenza; pneumonia caused by respiratory multinuclear virus (RSV); As well as, but is not limited to, A, B, or C.

Further examples of infectious viruses include: retroviridae; picornaviridae (eg, poliovirus, hepatitis A virus, enterovirus, human coxsackie virus, rhinovirus, echovirus); calciviridae (gastrointestinal) Togaviridae (eg equine encephalitis virus, rubella virus); Flaviviridae (eg dengue virus, encephalitis virus, yellow fever virus); Coronavirus (eg coronavirus); Rhabdovirus (Eg, vesicular stomatitis virus, rabies virus); Filoviridae (eg, Ebola virus); Paramyxovirus (eg, parainfluenza virus, mumps virus, measles virus, respiratory polynuclear virus); Tomyxoviridae (eg, influenza virus); Bunyaviridae (eg, Hantern virus, Bunyavirus, flavovirus, and Nairovirus); Arenavirus (haemorrhagic fever virus); Reoviridae (eg, reovirus, Orbi) Virnaviridae; Hepadnaviridae (Hepatitis B virus); Parvoviridae (Parvovirus); Papovaviridae (papilloma virus, polyomavirus); Adenoviridae (most adenoviruses) Virus); herpesviridae (herpes simplex virus (HSV) 1 and HSV-2, varicella-zoster virus, cytomegalovirus (CMV), poxviridae (smallpox virus, vaccinia virus, poxvirus)); And Iridoviridae (such as African swine fever virus); and unclassified viruses (eg causative agent of spongiform encephalopathy, δ hepatitis substance (considered to be a deficient satellite of hepatitis B virus), non A non-B hepatitis substance (class 1 = internal transmission; class 2 = parenteral transmission) (ie hepatitis C); Norwalk and related viruses, and astrovirus).

### Bacterial infections

The term "bacterial infection" as used herein includes the presence of bacteria in or on a subject that would benefit the subject if its growth is inhibited. Thus, in addition to referring to the presence of bacteria, the term "infection" also refers to unwanted resident flora.

The bacterial agent causing a bacterial infection may be a gram-positive bacterial agent selected from the group comprising, but not limited to, *Staphylococcus* spp, *Streptococci, Enterococcus* spp, *Leuconostoc* spp, *Corynebacterium* spp, *Bacillus* spp, *Anaerobic Cocci, Actinomyces* spp, *Clostridium* spp, *Nocardia* spp, *Listeria* spp, *Mycoplasma* spp, *Mycobacterium* spp.

The bacterial agent causing the bacterial infection may be a gram-negative bacterial agent selected from the group comprising, but not limited to, pathogenic *Escherichia coli*, Bacillus, *Pseudomonas, Helicobacter, Legionella, Neisseria gonorrhoeae, Neisseria meningitidis, Haemophilus influenzae*, *Klebsiella pneumoniae*, *Pseudomonas aeruginosa, Chlamydia trachomatis, Yersinia pestis, Plasmodium* (including *Plasmodium facliparum*) *and Trypanosoma* (including *Trypanozoma cruzi*).

### Sepsis

As it is well known in the art, sepsis, which is the leading cause of depth in critically ill patients, is defined as an infection-induced syndrome involving two or more of the following features of systemic inflammation : fever or hypothermia, leukocytosis or leukopenia, tachycardia, and tchypnea or supranormal minute ventilation. Sepsis encompasses systemic inflammatory response syndrome (SIRS), sepsis *per se*, severe sepsis, septic shock and multiple organ dysfunction syndrome (MODS). The skilled artisan may refer to Riedmann et al. (2003, J Clin Invets, Vol. 112 : 460-467) as well as to Hotchkiss et al. (2013, Nat Rev Immunol, Vol. 12 : 862-874).

In some embodiments, an antagonist antibody of human and macaque LILRB1 and/or LILRB2, or an antigen binding fragment thereof, is used as the sole active ingredient.

In some other embodiments, treatment of a disease or disorder with an antagonist antibody of human and macaque LILRB1 and/or LILRB2, or an antigen binding fragment thereof, also comprises subjecting the subject in need thereof to one or more further other treatments.

### Combination therapy

In embodiments, wherein the said disease or disorder is a proliferative disease, especially a cancer, the said treatment may further include another treatment selected from a group comprising radiotherapy treatment and chemotherapeutic treatment.

Thus, according to such other embodiments, the anti-cancer treatment with an antagonist antibody according to the present disclosure may also comprise the administration to the said subject of one or more further anti-cancer active ingredients.

An "anticancer agent" is defined as any molecule that can either interfere with the biosynthesis of macromolecules (DNA, RNA, proteins, etc.) or inhibit cellular proliferation, or lead to cell death by apoptosis or cytotoxicity for example. Among the anticancer agents, there may be mentioned alkylating agents, topoisomerase inhibitors and intercalating agents, anti-metabolites, cleaving agents, agents interfering with tubulin, monoclonal antibodies.

In embodiments, wherein the said disease or disorder is a disease caused by a bacterial infection, the said treatment may further be combined with a treatment with an anti-bacterial agent.

Thus, according to such other embodiments, a treatment with an antagonist antibody according to the present disclosure may also comprise the administration to the said subject of one or more further antibacterial active ingredients.

In embodiments, wherein the said disease or disorder is a disease caused by a viral infection, the said treatment may further be combined with a treatment with an anti-viral agent.

Thus, according to such other embodiments, a treatment with an antagonist antibody according to the present disclosure may also comprise the administration to the said subject of one or more further antiviral active ingredients.

### Compositions

The present disclosure provides compositions comprising an antagonist antibody, or an antigen binding fragment thereof, that binds both to human and macaque LILRB1 and/or LILRB2 as described herein.

As already stated elsewhere in the present disclosure, a given antagonist antibody, or an antigen binding fragment thereof, as described herein, when used as an active agent for a therapeutic treatment in human, may be identical to the candidate active agent that was used during early steps of clinical trials, especially during early steps performed in a primate model, since the said antagonist antibody binds both to macaque LILRB1 and LILRB2 receptors and to human LILRB2 and/or LILRB 1 receptors.

This disclosure also relates to pharmaceutical compositions comprising an antagonist antibody, or an antigen binding fragment thereof, that binds both to human and macaque LILRB 1 and/or LILRB2, as described herein, and a pharmaceutically acceptable carrier. The said pharmaceutical compositions comprise a therapeutically effective amount of an antagonist antibody, or an antigen binding fragment thereof, that binds both to human and macaque LILRB 1 and/or LILRB2 as described herein.

The term "therapeutically effective amount" refers to an amount of a drug effective to treat a disease or disorder in a mammal. In the case of cancer, the therapeutically effective amount of the drug may reduce the number of cancer cells; reduce the tumor size; inhibit (i.e., slow to some extent and preferably stop) cancer cell infiltration into peripheral organs; inhibit (i.e., slow to some extent and preferably stop) tumor metastasis; inhibit, to some extent, tumor growth; and/or relieve to some extent one or more of the symptoms associated with the disorder. To the extent the drug may prevent growth and/or kill existing cancer cells, it may be cytostatic and/or cytotoxic. For cancer therapy, efficacy in vivo can, for example, be measured by assessing the duration of survival, duration of progression free survival (PFS), the response rates (RR), duration of response, and/or quality of life.

Therapeutic formulations of the agents (e.g., antibodies) used in accordance with the present disclosure may be prepared for storage by mixing an antibody having the desired degree of purity with optional pharmaceutically acceptable carriers, excipients or stabilizers (Remington's Pharmaceutical Sciences 16th edition, Osol, A. Ed. (1980)), in the form of lyophilized formulations or aqueous solutions. Acceptable carriers, excipients, or stabilizers are nontoxic to recipients at the dosages and concentrations employed, and include buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes {e.g. Zn-protein complexes); and/or non-ionic surfactants such as Tween^{™}, Pluronics^{™} or polyethylene glycol (PEG).

The active ingredients may also be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nanoparticles and nanocapsules) or in macroemulsions. Such techniques are disclosed in Remington's Pharmaceutical Sciences 16th edition, Osol, A. Ed. (1980).

The formulations to be used for *in vivo* administration may be sterile. This is readily accomplished by filtration through sterile filtration membranes.

According to another particular aspect, the disclosure relates to a composition for use as a medicinal product in the prevention or treatment of a disease or disorder described herein, comprising an antagonist antibody, or an antigen binding fragment thereof, that binds both to human and macaque LILRB1 and/or LILRB2, in which the therapeutically effective quantity of the antibody administered to a patient is in a range from about 0.01 µg to about 30000 µg, preferably from about 0.1 µg to about 10000 µg, preferably from about 0.1 µg to about 5000 µg, preferably from about 0.1 µg to about 1000 µg, and more preferably from about 0.1 µg to about 100 µg.

In another aspect is provided herein a pharmaceutical kit comprising an antagonist antibody according to the present disclosure, or an antigen binding fragment thereof, the said kit comprising (i) a container that contains a composition comprising the said antagonist antibody, or antigen binding fragment thereof, and (ii) optionally informational material. The informational material may be descriptive, instructional, marketing or other material that relates to the therapeutic methods described herein and/or the use of the agents for therapeutic benefits.

In such a pharmaceutical kit, the container may be a bottle, a vial or a syringe that has optionally attached thereto the informational material in the form of a label.

### Detection/diagnostic

An antagonist antibody, or an antigen binding fragment thereof, that binds both to human and macaque LILRB1 and/or LILRB2 may be used for determining expression of LILRB1 and/or LILRB2 by cells or in tissue samples.

An antagonist antibody, or an antigen binding fragment thereof, that binds both to human and macaque LILRB1 and/or LILRB2 may also be used for determining the presence of circulating LILRB 1 and/or LILRB2 in body fluids, such as in blood samples or blood-derived samples, or lymphatic fluids. Blood derived samples encompass plasma and serum samples.

Determining expression of LILRB1 and/or LILRB2 by cells or in tissue samples encompass (i) detecting the presence of LILRB1 and/or LILRB2 and (ii) measuring an amount of LILRB1 and/or LILRB2 expressed by cells or present in a tissue sample. Most preferably, the presence or amount of LILRB1 and/or LILRB2 expressed by cells is detected or quantified at the cell surface, e.g. by immunohistochemical methods or by flow cytometry methods known in the art.

In some embodiments, as described elsewhere in the present specification, subjects are tested for determining whether their immune cells express LILRB 1 and/or LILRB2 at their surface, before performing a treatment with an an antagonist antibody, or an antigen binding fragment thereof, that binds both to human and macaque LILRB 1 and/or LILRB2 as described herein.

Thus, this disclosure also relates to an *in vitro* method for determining whether a subject which is affected with a disease or disorder selected in the group consisting of proliferative diseases, viral infections and bacterial infections, including sepsis, is eligible to a treatment with an antagonist antibody, or an antigen binding fragment thereof, that binds both to human and macaque LILRB 1 and/or LILRB2, wherein the said method comprises the steps of :
c) determining if tested cells from a sample previously obtained from the said patient express LILRB1 and/or LILRB2 at their membrane, and
d) concluding that the said patient is eligible to a treatment with an antagonist antibody, or an antigen binding fragment thereof, that binds both to human and macaque LILRB 1 and/or LILRB2 if membrane expression, or membrane over-expression, of LILRB1 and/or LILRB2 by the said tested cells has been determined at step c).

Step c) is performed by using an antagonist anti-LILRB1 and/or anti-LILRB2 antibody, or an antigen binding fragment thereof, the binding of which may be detected (i) by labeling the said antagonist anti-LILRB1 and/or anti-LILRB2 antibody with a detectable molecule or (ii) by using a secondary labeled antibody, according to well-known antibody detection techniques.

In preferred embodiments of the said *in vitro* method, it is concluded at step d) that the said patient is eligible (*i.e.* responsive) to a treatment with an antagonist antibody, or an antigen binding fragment thereof, that binds both to human and macaque LILRB 1 and/or LILRB2 when (i) a LILRB 1 and/or LILRB2 expression is determined at step c) and when (ii) the said LILRB 1 and/or LILRB2 expression level value is of a threshold reference value or more.

According to some preferred embodiments, determining cell surface expression of LILRB 1 and/or LILRB2 according to the said the method is performed by a immunohistochemical method or by a flow cytometry method.

According to some embodiments, determining the presence of LILRB 1 and/or LILRB2 in a body fluid previously obtained from a patient to be tested is performed according to an ELISA technique. For example, an antagonist antibody of Family AB1 or of Family AB2 is first immobilized on a support. The resulting functionalized support is incubated with a body fluid to be tested. Then, the presence of complexes between the immobilized antagonist antibodies and LILRB 1 and/or LILRB2 susceptible to be present in the tested body fluid is detected, *e*.*g*. by incubating the resulting preparation with a detectable molecule that binds to the complexed LILRB 1 and/or LILRB2. Such a detectable molecule may be another antibody that binds to LILRB 1 and/or LILRB2.

The cells that are used at step c) may originate from a biopsy tissue sample that has previously been collected from the tested subject, such as a biopsy cancer tissue sample that has previously been collected from the tested subject.

The present disclosure also provides means and methods for monitoring the level of expression of LILRB1 and/or LILRB2 in a subject with time.

Notably, the present disclosure provides means and *in vitro* methods for monitoring the level of expression of LILRB1 and/or LILRB2 in the course of a therapeutic treatment of a subject in need of the said therapeutic treatment, e.g. in the course of a therapeutic treatment of a subject affected with an immunosuppression state, a proliferative disease, a viral infection or a bacterial infection, including affected with a sepsis. These means and *in vitro* methods may allow monitoring the efficiency of a given therapeutic treatment, including monitoring the efficiency of a therapeutic treatment with an antagonist antibody according to the present disclosure, or an antigen binding fragment thereof.

The present disclosure also pertains to an *in vitro* method for monitoring the level of expression of a LILRB1 and/or a LILRB2 receptor in a subject comprising the steps of :
a) determining the level of expression of LILRB1 and/or LILRB2 in a sample previously obtained in a subject by using an antagonist antibody, or an antigen binding fragment thereof, that binds both to human and macaque LILRB 1 and/or LILRB2 according to the present disclosure,
b) comparing the level of expression of LILRB1 and/or LILRB2 determined at step a) with the level of expression of LILRB1 and/or LILRB2 that has been determined in a sample originating from the same subject and that has been obtained earlier than the sample used at step a).

The present disclosure further relates to an *in vitro* method for monitoring the efficiency of a therapeutic treatment in a subject, the said method comprising steps a) and b) of the monitoring method above and further comprising the following step :
c) determining that the said therapeutic treatment is efficient if the level of expression of LILRB 1 and/or LILRB2 determined at step a) is lower than the level of expression of LILRB 1 and/or LILRB2 that has been determined in a sample originating from the same subject and that has been obtained earlier than the sample used at step a).

The present disclosure also provides a kit for determining the presence of, or the level of expression of, LILRB 1 and/LILRB2, the said kit comprising :
a) an antagonist antibody, or an antigen binding fragment thereof, that binds both to human and macaque LILRB1 and/or LILRB2 as disclosed herein, and
b) one or more reagents for detection or diagnosis.

### Sequences

Sequences useful according to the present disclosure are gathered in the Table below.

**Table 1 : Sequences**

| **SEQ ID NO.** | **Sequence** | **Description** |
|---|---|---|
| 1 | GFTFNTHA | B 1.2.1. antibody, H-CDR1 |
| 2 | IRSKSSNYAT | B 1.2.1. antibody, H-CDR2 |
| 3 | VMVYYGNSPYYSMDY | B 1.2.1. antibody, H-CDR3 |
| 4 | EVQLVESGGGLVQPKGSLKLSCAASGFTFNTHAMHWVRQ APGKGLEWVARIRSKSSNYATFYADSVKDRFTISRDDSQSM LYLQMNNLKTEDTAIYFCVMVYYGNSPYYSMDYWGQGTS VTVSS | B1.2.1.antibody, variable domain heavy chain |
| 5 | ENVGTY | B 1.2.1. antibody, L-CDR1 |
| 6 | GAS | B 1.2.1. antibody, L-CDR2 |
| 7 | GQSYSSPLT | B 1.2.1. antibody, L-CDR3 |
| 8 | NIVMTQSPKSMSMSVGERVTLSCKASENVGTYVSWYQQK PEQSPKLLIYGASNRYTGVPDRFTGSGSATDFTLIINNIQAE DLADYHCGQSYSSPLTFGAGTKLELK | B1.2.1.antibody, variable domain light chain |
| 9 | GFTFSDYG | B1.2.2. antibody, H-CDR1 |
| 10 | ISSGSSTS | B1.2.2. antibody, H-CDR2 |
| 11 | ARPGSNWDVYYAMDY | B1.2.2. antibody, H-CDR3 |
| 12 | EVQLVESGGGLVKPGGSLKLSCAASGFTFSDYGMHWVRQ APEKGLEWVAYISSGSSTSYYADTVKGRFTISRDDAKNTLFL QMTSLRSEDTAMYYCARPGSNWDVYYAMDYWGQGTSV TVSS | B1.2.2. antibody, variable domain, heavy chain |
| 13 | QSVSTSSYSY | B1.2.2. antibody, L-CDR1 |
| 14 | NAS | B1.2.2. antibody, L-CDR2 |
| 15 | QHSWEIPFT | B1.2.2. antibody, L-CDR3 |
| 16 | DIVLTQSPASLAVSLGQRATISCRASQSVSTSSYSYMHWYQ QKPGQPPKLLIKNASNLESGVPARFSGSGSGTDFTLNIHPV EEEDTATYYCQHSWEIPFTFGGGTKLEIK | B1.2.2. antibody, variable domain, light chain |
| 17 | DYKDDDDK | Flag sequence |
| 18 | MSALLILALVGAAVA | Signal peptide |
| 19 | RYPLTFGWCY | MHC-I A*2402 HIV-1 Nef 134-143 |
| 20 | GEIYKRWII | MHC-I B*0801 HIV-1 Gag 259-267 |
| 21 | GPRKPIKCW | cynomolgus macaque Mafa-A1*063 SIV gag GW9 |

In case of discrepancy(ies) between the sequences described in the present disclosure and those listed in an appended sequence listing, the correct sequences are those described in the present disclosure.

The present disclosure is further illustrated by, without in any way being limited to, the examples herein.

### EXAMPLES

### A. Materials and Methods of the examples

### A.1. Animals

Cynomolgus macaques were housed in the facilities of the Infectious Disease Models and Innovative Therapies (IDMIT) Center, part of the "Commissariat a l'Energie Atomique et aux Energies Alternatives" (CEA, Fontenay-aux-Roses, France). Non-human primates (NHP) were used in accordance with French national regulations, under the supervision of national veterinary inspectors (CEA Permit Number D 92-032-02). The CEA complies with the Standards for Human Care and Use of Laboratory Animals, of the Office for Laboratory Animal Welfare (OLAW, USA) under OLAW Assurance number #A5826-01. The use of NHP at the CEA is in conformity with the recommendations of European Directive (2010/63, recommendation No. 9). The animals were used under the supervision of the veterinarians in charge of the animal facility. These studies were approved and accredited by the ethics committee "Comité d'Ethique en Experimentation Animale du CEA".

Biozzi mice were bred at the animal care unit of the CEA (Gif sur Yvette, France). All experiments were performed in compliance with French and European regulations on the care of laboratory animals (European Community Directive 86/609, French Law 2001-486, 6 June 2001) and with the agreements of the Ethics Committee of the Commissariat a l'Energie Atomique (CEtEA 'Comité d'Ethique en Experimentation Animale' n° 44) no. 12-026 and 15-055 delivered to S. S. by the French Veterinary Services and CEA agreement D-91-272-106 from the Veterinary Inspection Department of Essonne (France).

### A.2. Cynomolgus macaque LILR sequencing and cloning

For cynomolgus macaque LILRs (mLILRs) sequencing, PBMCs from one SIV⁻ and one SIV⁺ cynomolgus macaques were isolated and CD14⁺ cells were sorted using monocytes miltennyi microbeads kit and then stimulated with LPS for 4 h. RNAs from CD14⁺ cells were extracted by RNeasy mini kit (Qiagen). After mRNA isolation and reverse transcription cDNA were sequenced using a next-generation sequencing approach (MiSeq, Illumina). mLILRs full-length sequences from RNA sequencing were reconstructed in silico using rhesus macaque LILRs references and various softwares including Bowtie, GAP4 and Geneious version 8.1.4 (Biomatters Ltd.). Multiple mLILRs genes were identified and specific sets of primers (Eurofins Genomics) targeting the different mLILR cDNAs were designed. For mLILRs cloning, total RNA was extracted from freshly purified PBMCs of 5 cynomolgus macaques using RNeasy Mini Kit (Qiagen). The RNA was reversely transcribed into cDNA using SuperScript VILO (Invitrogen). Amplification of mLILRs was done using the specific synthesized primers (Eurofins Genomics) and the Platinum PCR SuperMix (Invitrogen). The parameters for PCR were 95°C for 4 min followed by 40 cycles at 94°C for 30 sec, 58°C for 30 sec, 72°C for 2 min and finally at 72°C for 10 min. TOP10 competent bacteria were transformed with the resulting PCR products using TOPO TA Cloning KIT (Invitrogen). Sequencing of cDNAs were carried-out by Eurofins Genomics. The sequencing results were analyzed with Geneious version 8.1.4 (Biomatters Ltd.) software.

### A.3. Cell lines production

As no commercially available antibody is able to recognize the different members of the mLILRs family, a flag (DYKDDDDK - SEQ ID NO. 17) sequence was added to the N-terminal part of the full-length mLILRs-encoding cDNAs. Signal peptide of mLILRs was removed and replaced by a new signal peptide (MSALLILALVGAAVA - SEQ D NO. 18) to ensure efficient surface expression of the receptors. The modified mLILRs cDNA sequences were synthesized and subcloned into the lentiviral vector pLenti-GIII-CMV-GFP-2A-Puro (ABM Inc.) by ProteoGenix (Schiltigheim, France). Plasmid constructions were amplified through STLB2 competent bacteria (Invitrogen) transformation and delivered using NucleoBond^{™} Xtra Midi Kit (Macherey-Nagel). For generation of the different mLILR cell lines, mLILRs cDNA-encoding plasmids were co-transfected with pLenti-P2A packaging and pLenti-P2B envelope lentiviral plasmids (ABM Inc.) into HEK293T cells using TransIT^{®}-293 (Mirus, EuroMedex), according to the manufacturer's instructions. Transfection efficiency was evaluated by the level of GFP expressed by HEK293T transfected cells. Stably transfected cells were selected with Puromycin 4µg/ml (Gibco) for one week and maintained in complete medium (DMEM (Gibco), 10% FBS (Eurobio), 1% Penicillin-Streptomycin-Neomycin (PSN) antibiotic mixture (Gibco). Receptor expression on the surface of each HEK293T-mLILR cell line produced was confirmed by flow cytometry using an anti-DYKDDDDK antibody (clone REA213) from Miltenyi Biotec. Cell lines highly co-expressing mLILRs and GFP were sorted using the Becton Dickinson (BD) FACSAria I cell sorter and maintained in complete medium. M8 HLA-G1 cell line was produced by transfecting M8 melanoma cells with pcDNA3.1 vector containing HLA-G1 cDNA by electroporation. CHO cell lines expressing either human LILRB1 or LILRB2 were kindly donated by Donald Shaffer (Jounce Therapeutics Inc., Cambridge, USA).

### A.4. Cynomolgus macaque LILR-Fc fusion proteins production

The mLILR-Fc fusion proteins were produced and purified in collaboration with the team of Gerard and Sandy Zurawski (Baylor Research Institute, Dallas, USA). The cDNAs encoding the extracellular domain of the different mLILRs were fused to a hIgG1-Fc by molecular biology for the generation of mLILR-Fc fusion proteins expressing the Fc region (CH2 and CH3 domains) of the human IgG1 heavy chain and the hinge region. The plasmid constructions were transfected into HEK293T cells to produce the mLILR-Fc soluble proteins with optimal glycosylation. Each mLILR-Fc fusion protein secreted into the culture supernatants was harvested and purified by affinity chromatography.

### A.5. B1.2.1 and B1.2.2 monoclonal antibody production

Monoclonal antibodies (mAbs) were raised in *Biozzi* mice by immunizing with mLILRB1.2-Fc or mLILRB2.1-Fc (30 µg per injection) fusion protein. Mice eliciting the highest anti-mLILRB1.2-Fc or mLILRB2.1-Fc antibody response were given an intravenous boost injection 3 days before being sacrificed for splenic B cell fusion, according to Köhler and Milstein (Kohler & Milstein, 1975). Hybridoma culture supernatants were screened for antibody production, specificity for mLILRB1.2 and mLILRB2.1, cross-reactivity for hLILRB1 and hLILRB2 and blocking capacity, by either enzyme immunoassay, or either flow cytometry, or either both methods (see below for details). Selected hybridomas were subsequently cloned by limiting dilution. Monoclonal antibodies were produced in hybridoma supernatants and further purified using AKTA protein purifier. Purity of mAbs was assessed by SDS-PAGE in reducing and nonreducing conditions using an Agilent 2100 Bioanalyser. Endotoxin levels in purified mAbs were determined by Pierce LAL Chromogenic Endotoxin Quantitation Kit (Thermo Fisher Scientific) according to the manufacturer's protocol.

### A.6. Immunoenzymatic screening of anti-LILRB1 and -LILRB2 hybridoma supernatants on mLILR-Fc

Enzyme immunoassays (EIA) were performed by transfering 50 µl of diluted hybridoma culture supernatants into 96-well, flat-bottom microplates (MaxiSorp^{™} immunoplate, Nunc) coated with goat anti-mouse immunoglobulin antibody (Jackson ImmunoReseach), and incubated overnight at 4 °C. After washes, biotinylated mLILRB1.2-Fc or mLILRB2.1-Fc fusion proteins (50 ng/ml) were added (100 µl per well) and plates were reacted for 1 h at room temperature (RT). Plates were washed and reacted for 30 min at RT with 100 µl per well of 1 EU/ml of acetylcholinesterase (AChE)-labeled streptavidin. After several washes, AChE activity was revealed by Ellman's colorimetric method (Grassi et al., 1989) by measuring absorbances at 414 nm after 1 h. All reagents were diluted in EIA buffer (0.1 M phosphate buffer [pH 7.4] containing 0.15 M NaCl, 0.1% BSA, and 0.01% sodium azide). Plates coated with proteins were saturated in EIA buffer (18 h at 4°C) and washed with washing buffer (0.1 M potassium phosphate [pH 7.4] containing 0.05% Tween 20).

### A. 7. Flow cytometry screening of anti-LILRB1 and -LILRB2 hybridoma supernatants and mAbs on mLILR and hLILR cell lines

Flow cytometry screenings were performed using 0,2.10⁶ cells of each HEK293T mLILRs cell lines, CHO hLILRB1 or hLILRB2 cell lines, placed into 96-well, cell culture-treated, U-shaped-bottom microplate (Falcon^{™}, Thermo Fisher Scientific) and stained with LIVE/DEAD viability dye (Invitrogen) for 15 min at RT to exclude dead cells. After a wash with DPBS IX (Gibco), cells were incubated with 50 µl of hybridoma supernatants or 10 µg/ml monoclonal antibodies for 15 min at RT in qs 100 µl DPBS IX to assess antibodies specificity and cross-reactivity. Cells were washed and incubated for 15 min at RT with an affinipure secondary goat anti-mouse or -human IgG (H+L) antibody conjugated to the Alexa Fluor (AF) 647 dye (Jackson ImmunoResearch) in qs 100 µl DPBS IX. Finally, cells were washed and fixed with BD CellFIX IX and acquired on BD LSR Fortessa cytometer. Purified mouse IgG1 κ isotype control from Becton Dickinson and purified IgG1 mAb produced in house were used as IgG isotype control.

### A.8. Immunoenzymatic screening of B1.2.1 and B1.2.2 mAbs on hLILR-Fc

Enzyme immunoassays (EIA) were performed by transferring 5 µg/ml of hLILR-Fc, purchased from R&D Systems, into 96-well, flat-bottom microplates (MaxiSorp^{™} immunoplate, Nunc) coated with goat anti-human immunoglobulin antibody (Jackson ImmunoReseach), and incubated overnight at 4 °C. After washes, plates were saturated with EIA buffer for 3 h at RT. Plates were washed and several diluted concentration of 10 µg/ml monoclonal antibodies (10, 3.3, 1.1, 0.37, 0.12, 0.041 and 0.014 µg/ml) at a volume of 100 µl per well were added for 2 h incubation at RT. After washes, plates were reacted for 1 h at RT with 100 µl per well of 2 EU/ml of acetylcholinesterase (AChE)-labeled goat anti-mouse antibody produced in house, then washed again. AChE activity was revealed by Ellman's colorimetric method by measuring absorbances at 414 nm after 1 h and 40 min.

### A.9. Affinity determination of B1.2.1 and B1.2.2 mAbs

The affinities of B1.2.1 and B1.2.2 mAbs for the different human and macaque LILRs-Fc fusion proteins were determined by Bio-layer Interferometry using the ForteBio system (Pall Laboratory). B1.2.1 and B1.2.2 mAbs prepared at 10µg/ml in EIA buffer + 0.02% Tween 20 (Sigma-Aldrich) were dispensed in 96-well microplate at a volume of 200 µl per well. The same concentration occupied 8 vertical wells. In another wells, LILR-Fc fusion proteins were each dispensed at 8 titrated concentrations. A glycine (Sigma-Aldrich, pH [1.4]) regeneration solution and EIA buffer + 0.02% Tween 20 for baseline stabilization and neutralization was also prepared. The plate was agitated at 1000 rpm over the entire course of the experiment. Prior to the binding measurements, the anti-mouse Fc (AMC) sensors tips were hydrated in EIA buffer + 0.02% tween 20. The sensor tips were then transferred to the EIA buffer + 0.02% Tween 20 for the baseline, after mAb - containing wells for 300 sec loading step. After baseline step in EIA buffer + 0.02% Tween 20 for 60 sec, the binding kinetics were measured by dipping the mAb-coated sensors into the wells containing LILR-Fc fusion protein at varying concentrations. The binding interactions were monitoring over 900 sec associated period and followed by a 900 sec dissociation period in the wells containing EIA buffer +0.02 % Tween 20. The AMC sensors tips were regenerated with wells containing glycine [pH1.4] and neutralized in the EIA buffer + 0.02% tween 20 between each binding cycle. The equilibrium dissociation constant (K_{D}) was calculated using the ratio between the dissociation rate constant (k_{off}) and the association rate constant (kₒₙ), obtained with global Langmuir 1:1 fit (Octet Data Analysis software, vHT.10).

### A.10. Blocking tests

Blocking tests of soluble hLILRB1/B2-Fc fusion proteins interaction with M8 HLA-G1 cell line were performed by incubating 5 µg/ml of fusion proteins with either 50 µl of hybridoma supernatants or either 10 µg/ml of purified B1.2.1 or B1.2.2 mAbs for 20 min at RT in qs 100 µl DPBS IX. 0,2.10⁶ M8 HLA-G1 cells were placed into 96-well, cell culture-treated, U-shaped-bottom microplate and stained with LIVE/DEAD viability dye. After a wash, cells were incubated with mixes of fusion proteins with mAbs for 20 min at RT. Cells were washed to remove the unbound fusion proteins and incubated for 15 min at RT with an affinipure secondary goat anti-human IgG (H+L) antibody conjugated to the AF647 dye (Jackson ImmunoResearch) at a volume of qs 100 µl DPBS IX per well. Cells were finally washed and fixed with BD CellFIX IX and acquired on BD LSR Fortessa cytometer.

Same protocol was performed for the blocking test of hLILRBB2-Fc fusion protein interaction with M8 HLA-G1 FHC cell line. A preincubation step of 8.10⁶ M8 HLA-G1 cells with 1 000 µl of HCl Glycin (300mM) buffer (pH=3) with BSA 1% was done for 2 min at RT. After two washes with 50 ml FBS stain buffer (BD), 0.4.10⁶ M8 HLA-G1 treated cells were placed into 96-well, cell culture-treated, U-shaped-bottom microplate for the blocking tests. Complete dissociation of the beta-2-microglobulin from the HLA-G1 heavy chain was confirmed by staining the cells with MHC-I free chain monoclonal antibody (clone A4) conjugated to APC from eBioscience. Purified isotype control IgG1 mAb produced in house was used as IgG isotype control.

For blocking tests between membrane bound LILRs and soluble MHC-I ligand, CHO hLILRB1 cell line interaction with human MHC-I A*2402 HIV-1 Nef 134-143 (RYPLTFGWCY - SEQ ID NO. 19) pentamer (ProImmune), CHO hLILRB2 cell line interaction with human MHC-I B*0801 HIV-1 Gag 259-267 (GEIYKRWII - SEQ ID NO. 20) pentamer (ProImmune), and niLILRB1.2 or mLILRB2.1 cell line interactions with cynomolgus macaque Mafa-A1*063 SIV gag GW9 (GPRKPIKCW - SEQ ID NO. 21) - APC tetramer (MBL International), were used. 2.10⁵ cells from each cell lines expressing human or cynomolgus macaque LILRs were placed into 96-well, cell culture-treated, U-shaped-bottom microplate and stained with LIVE/DEAD viability dye. After a wash, cells were incubated with either 50 µl of hybridoma supernatants or either 10 µg/ml of B1.2.1 or B1.2.2 mAbs in qs 100 µl DPBS IX for 15 min at RT. Cells were washed to remove the unbound antibodies and incubated for 30 min at RT with pentamers or APC-conjugated tetramers in a total volume of qs 100 µl DPBS IX per well. After a wash, cells incubated with pentamers were stained with a secondary Pro5 Fluorotag antibody conjugated to APC (ProImmune) in qs 100 µl DPBS IX. Cells were finally washed and fixed with BD CellFIX IX and acquired on BD LSR Fortessa cytometer. Purified IgG1 B1.2 mAb produced in house and displaying no blocking capacity was used as IgG isotype control.

### A.11. B1.2.1 and B1.2.2 mAbs distribution on PBMCs

Human and cynomolgus macaque PBMCs previously purified by Ficoll gradient and frozen in FBS 10% dimethyl sulfoxide (Sigma-Aldrich) were thawed in RPMI (Gibco), 10% FBS, 1% PSN. 2.10⁶ cells were placed into 5mL round bottom polystyrene fACS tubes (Falcon^{™}, Thermo Fisher Scientific) and stained with LIVE/DEAD viability dye. After a wash, cells were incubated for 15 min RT with chicken anti-CADMl (clone 3E1) mAb from MBL Life Science. Cells were washed and stained for 30 min at 4°C with an antibody mix containing either AF647-conjugated B1.2.1 mAb, either AF647-conjugated B1.2.2 mAb or either AF647-conjugated mouse IgG1 κ isotype control (Becton Dickinson). Cells were finally washed and fixed with BD CellFIX IX and acquired on BD LSR Fortessa cytometer.

Antibody mixes for human and cynomolgus macaque PBMCs staining contained anti-CD14-BV711 (clone M5E2), anti-CD11c-BV650 (clone 3.9) and anti-CD1c-APC-Cy7 (clone L161) from BioLegend. Anti-CD3-V450 (clone SP34-2), anti-CD20-V450 (L27), anti-CD123-PerCP-Cy5.5 (clone 7G3) and anti-HLA-DR-PE-Cy7 (clone L243) from Becton Dickinson. Anti-CD16-PE/Dazzle 594 (clone 3G8) from Sony Biotechnology. But also, donkey anti-chicken-PE from Jackson ImmunoResearch and anti-CD45-V500 (clone HI30 for human or clone D058-1283 for macaque PBMCs staining) from Becton Dickinson.

### Example 1 : Generation of anti-LILRB1 and anti-LILRB2 monoclonal antibodies that cross-react in cynomolgus macaque and humans.

The cDNA corresponding to cynomolgus macaques LILRs (mLILRs) were cloned from PBMCs from cynomolgus macaques hosted at IDMIT non-human primate facility (CEA Fontenay aux roses, France). Comparison of cDNA LILR sequences from human and cynomolgus macaque indicated that mLILRs and human LILRs show similar structure. Among cynomolgus macaques assessed we could identify ten mLILRs including five activating mLILRA (A1-A5) and five inhibitory mLILRB (LILRB1-B5) members. According to sequence alignment, we defined mLILRB1 and mLILRB2 as the cynomolgus macaque equivalent of human LILRB1 (hLILRB1) and human LILRB2 (hLILRB2), respectively. Using the characterized mLILR sequences, mLILRB1.2-Fc and mLILRB2.1-Fc fusion proteins were produced and used to immunized mice to develop anti-mLILRBl and anti-mLILRB2 monoclonal antibodies (mAbs). Hybridoma supernatants (HS) were screened for mLILRB1.2-Fc and mLILRB2.1-Fc binding by ELISA. HS specific for the Fc domain of the fusion proteins were removed following a flow cytometry screening on cell lines stably expressing mLILRB1 and mLILRB2. Next, HS were screened and selected for their cross-reactivity on CHO cell lines expressing either hLILRB1 or hLILRB2. Finally, HS were selected for their capacity to block in *vitro* the LILRB1 and/or LILRB2 interaction with their human and macaque MHC-I ligands. At the end of the different screening, two hybridomas from the mLILRB1.2-Fc immunization were selected and purified to obtain the B1.2.1 and B1.2.2 blocking mAbs. The graphical representation and the sequences of these two mAbs' variable domains are depicted in **Figure 5**.

### Example 2 : B1.2.1 and B1.2.2 monoclonal antibodies cross-react with both human and cynomolgus macaque LILRs.

Because members of the LILR family share a high degree of homology, we tested the B1.2.1 and B1.2.2 mAbs for their potential cross-reactivity on each human and cynomolgus macaque LILRs (**Figure 1**). Using human soluble LILR-Fc fusion proteins we showed that B1.2.1 interacts with both hLILRB1 and hLILRB2 whereas B1.2.2 interacts with hLILRB1 and weakly with hLILRB2 (**Figure 1A**). Our results also showed that both B1.2.1 and B1.2.2 mAbs cross-react with hLILRA1 andhLILRA3. The interactions of B1.2.1 and B1.2.2 mAbs with inhibitory receptors hLILRB1 and hLILRB2 expressed on transduced CHO cell lines was confirmed by flow cytometry analysis (**Figure 1B**). To assess the cross reactivity of B1.2.1 and B1.2.2 mAbs with cynomolgus macaque LILRs we generated cell lines expressing each mLILR. Our results show that both B1.2.1 and B1.2.2 mAbs cross-react with the two mLILRB1 alleles and with mLILRA1 allele 3 (**Figure 1C**). Moreover, B1.2.1 mAb only interacts with mLILRB2 allele 2, whereas B1.2.2 mAb interacts with the two mLILRB2 alleles. We could not assess the reactivity of the mAb with mLILRA3. Finally, binding affinity of B1.2.1 and B1.2.2 mAbs with human and macaque LILRs were determined using biolayer interferometry (**Table 2**). The equilibrium dissociation constant (K_{D}) affinities were calculated in the range of 1.3 10⁻⁹ - 8.7 10⁻¹⁰ M for hLILRs-Fc and 2.9 10⁻⁹- 3.9 10⁻¹² M for mLILRs-Fc.

### Example 3 : B1.2.1 and B1.2.2 monoclonal antibodies block LILRB1 and LILRB2 interactions with classical and non-classical MHC-I ligands.

LILRB 1 and LILRB2 exhibit various profiles of interactions and affinity levels with membrane and soluble ligands (Burshtyn & Morcos, 2016). Among these ligands, LILRB1 and LILRB2 can bind to all of the classical human leukocyte antigen class I (HLA-I) molecules (HLA-A, -B and -C) and some of the non-classical HLA-I (HLA-G and -F) (Jones et al., 2011; Lepin et al., 2000; Navarro et al., 1999; Shiroishi, Kuroki, Rasubala, et al., 2006). In order to investigate the blocking capacity of purified B1.2.1 and B1.2.2 mAbs on human LILR / MHC-I interactions, we first used HLA-I pentamers harboring HIV peptides and displaying high level of interaction with hLILRB1 and hLILRB2 expressed on cell lines (**Figure 2A**). We found that B1.2.1 mAb was able to block the interaction of HLA-I pentamer with hLILRB2 but not with hLILRB1. Moreover, B1.2.2 mAb completely blocked the interaction of HLA-I pentamers with hLILRB1 and had no effect on hLILRB2. To confirm these results, we employed another interaction system using hLILRB 1-Fc and hLILRB2-Fc soluble fusion proteins and M8-cell line expressing the non-classical HLA-G1 molecule (**Figure 2B**), a ligand preferentially recognize by these two LILRs (Shiroishi, Kuroki, Rasubala, et al., 2006; Wang et al., 2019). Our results indicated that B1.2.1 mAb partially blocked hLILRB2-Fc interaction with HLA-G1 whereas B1.2.2 mAb completely blocked hLILRB 1-Fc / HLA-G1 interaction. For both interaction system, the lack of blocking activity from B1.2.2 on hLILRB2 results from it poor binding capacity to bind this receptor. To determine the blocking capacity of the purified monoclonal antibodies on mLILRB1 or mLILRB2 / MHC-I interactions in cynomolgus macaque, we first set up a binding assay between a cynomolgus macaque (*Macaca fascicularis*; Mafa) allele A1*063 MHC-I tetramer harboring a SIV peptide with niLILRB1.2 or mLILRB2.1 expressed on cell lines (**Figure 2C**). Thank to this approach, we could demonstrate that both B1.2.1 and B1.2.2 mAbs blocked Mafa-A1*063-MHC-I interaction with mLILRB1.2 cell line. B1.2.2 mAb also partially blocked the interaction of Mafa-A1*063-MHC-I tetramer with mLILRB2.1 cell line. Collectively, the results indicate that B1.2.1 is a blocking mAb for human LILRB2 and cynomolgus macaque LILRB 1.2 whereas B 1.2.2 is a blocking mAb for human LILRB 1 and cynomolgus macaque LILRB 1.2 and to a lesser extent for mLILRB2.1. These results although highlight that macaque LILRB 1.2 allele share structural proximity with both human LILRB 1 and LILRB2.

### Example 4 : B1.2.1 mAb blocks the interaction of human LILRB2 with HLA-class I free heavy chain molecules.

HLA-I class molecules are composed of a heavy chain including three domains (α1,-α2,α3) non-covalently associated with beta-2-microglobulin (B2M) subunit. Structural studies previously showed that LILRB 1 and LILRB2 interact via their D1 domain with the α3 domain of HLA-class I heavy chain while their D1 and D2 domains interact with the B2M subunit (Shiroishi, Kuroki, Rasubala, et al., 2006; Wang et al., 2019; Willcox et al., 2003). LILRB2 has a greater interacting interface with the α3 domain, while LILRB 1 strongly depends on the interaction with B2M to form a stable complex. Consequently, LILRB2 can interact with the HLA-I heavy chain associated or not with B2M whereas LILRB 1 binds exclusively with the HLA-I complexes associated with B2M (Jones et al., 2011; Shiroishi, Kuroki, Rasubala, et al., 2006). Because LILRB2 is able to interact with the B2M-free HLA class I heavy chain (FHC), we investigated if the generated antibodies were able to block this interaction. For this purpose, HLA-G1 FHC expressing cells were generated by removing B2M molecules from the surface of M8-HLA-G1 cells with an acid treatment. The efficiency of this treatment to induce FHCs on M8-HLA-G1 cells was verified using an antibody recognizing specifically MHC-I molecules dissociated from B2M (MHC-I FHC). As shown in **Figure 3A**, after acid treatment all M8-HLA-G1 cells were expressing high level of FHCs. Concordantly with previous study (Shiroishi, Kuroki, Ose, et al., 2006), LILRB2-Fc was still interacting with M8-HLA-G1 FHC despite the absence of B2M (**Figure 3B**). Moreover, B1.2.1 mAb completely blocked the interaction of hLILRB2-Fc with HLA-G1 FHC. In agreement with it poor binding to hLILRB2, no blocking activity was observed for B 1.2.2 mAb (**Figure 3B**).

### Example 5 : B1.2.1 and B1.2.2 monoclonal antibodies display similar distribution on human and macaque myeloid cells.

LILRB1 and LILRB2 are mainly found on immune subsets from the myeloid lineage (van der Touw et al., 2017). Therefore, we evaluated and then compared binding and distribution of the mAbs on human and macaque monocytes and dendritic cells subsets by flow cytometry analysis Stainings of PBMCs with fluorescent B1.2.1 and B1.2.2 mAbs showed similar distribution among myeloid cell subsets with a high expression of LILRB 1 and LILRB2 on each human monocyte subsets (classical, intermediate and non-classical monocytes) and lower expression on human cDC2 and pDC subsets (**Figure 4A**). Interestingly, staining of myeloid cells with B1.2.1 mAb was higher than with B1.2.2 mAb. This difference could result from the absence of hLILRB2 recognition by B1.2.2 compared to B1.2.1 (**Figure 1A-B**). Since peripheral blood cDC1s usually do not express LILRB1 or LILRB2 in resting conditions (Colletti et al., 2016), we use this cell subset as negative control of B1.2.1 and B1.2.2 staining. In agreement with previous studies, none of the mAbs stained cDC1s. Staining of cynomolgus macaque PBMCs with B1.2.1 and B1.2.2 mAbs showed similar cell subset distributions to that observed on human (**Figure 4B**). Unlike human monocytes and DCs, B1.2.2 mAb expression level was higher on macaque myeloid cells than B1.2.1. This expression level could result from the broader recognition range of B1.2.2 mAb on macaque LILRs (**Figure 1C**).

**Table 2 : Binding affinity of B1.2.1. and B1.2.2. to various LILR receptors**

| | Human | | | | Cynomolgus macaque | | | | |
|---|---|---|---|---|---|---|---|---|---|
| | **hLILRA1** | **hLILRA3** | **hLILRB1** | **hLILRB2** | **mLILRA1.3** | **mLILRB1.1** | **mLILRB1.2** | **mLILRB2.1** | **mLILRB2.2** |
| **B.1.2.1.** kD (M) | 7.3 x 10⁻¹⁰ | 1.3 x 10⁻⁹ | 5.3 x 10⁻¹⁰ | 1.4 10⁻¹⁰ | ND* | 3.9 x 10⁻¹² | 1.4 x 10⁻¹¹ | NB** | 2.9 x 10⁻⁹ |
| **B1.2.2.** KD (M) | 3.7 x 10⁻¹⁰ | 3.2 x 10⁻⁹ | 8.7 x 10⁻¹⁰ | NB | ND | 3.6 x 10⁻⁹ | 1.2 x 10⁻¹⁰ | 4.8 x 10⁻¹⁰ | 7.1 x 10⁻¹¹ |

| | | | | | | | | | |
|---|---|---|---|---|---|---|---|---|---|
| ** ND : Not Determined* ** *NB : No Binding* | | | | | | | | | |

## Claims

1. An antagonist antibody, or an antigen binding fragment thereof, that binds both to human and macaque LILRB 1 and/or LILRB2, wherein the said antagonist antibody is selected from the group consisting of:
A) an antagonist antibody "AB1", or an antigen binding fragment thereof, comprising :
(a) a heavy chain wherein the variable domain comprises :
- a H-CDR1 having a sequence set forth as SEQ ID NO. 1,
- a H-CDR2 having a sequence set forth as SEQ ID NO. 2, and
- a H-CDR3 having a sequence set forth as SEQ ID NO. 3, and/or
(b) a light chain wherein the variable domain comprises
- a L-CDR1 having a sequence set forth as SEQ ID NO. 5,
- a L-CDR2 having a sequence set forth as SEQ ID NO. 6, and
- a L-CDR3 having a sequence set forth as SEQ ID NO. 7,
and
B) an antagonist antibody "AB2", or an antigen binding fragment thereof, comprising :
(a) a heavy chain wherein the variable domain comprises :
- a H-CDR1 having a sequence set forth as SEQ ID NO. 9,
- a H-CDR2 having a sequence set forth as SEQ ID NO. 10, and
- a H-CDR3 having a sequence set forth as SEQ ID NO. 11, and/or
(b) a light chain wherein the variable domain comprises :
- a L-CDR1 having a sequence set forth as SEQ ID NO. 13,
- a L-CDR2 having a sequence set forth as SEQ ID NO. 14, and
- a L-CDR3 having a sequence set forth as SEQ ID NO. 15.

2. The antagonist antibody "AB1", or antigen binding fragment thereof, according to claim 1, comprising a heavy chain variable domain having at least 85% amino acid identity with SEQ ID NO. 4.

3. The antagonist antibody "AB1", or antigen binding fragment thereof, according to claim 1, comprising a light chain variable domain having at least 85% amino acid identity with SEQ ID NO. 8.

4. The antagonist antibody "AB2", or antigen binding domain fragment thereof, according to claim 1, comprising a heavy chain variable domain having at least 85% amino acid identity with SEQ ID NO. 12.

5. The antagonist antibody "AB2", or antigen binding fragment thereof, according to claim 1, comprising a light chain variable domain having at least 85% amino acid identity with SEQ ID NO. 16.

6. The antagonist antibody according to claim 1, which is a chimeric antibody or a humanized antibody.

7. The antagonist antibody, or antigen binding fragment thereof, according to any one of claims 1 to 6, which is selected from the group consisting of Fv, Fab, F'ab')2, dsFv, scFv, sc(Fv)2 and diabodies.

8. A nucleic acid comprising a sequence encoding an antagonist antibody, or antigen binding fragment thereof, according to any one of claims 1 to 7.

9. A vector comprising a nucleic acid according to claim 8.

10. A host cell comprising a nucleic acid according to claim 8 or a vector according to claim 9.

11. A pharmaceutical composition comprising an antagonist antibody according to any one of claims 1 to 7 and a pharmaceutically acceptable carrier.

12. The antagonist antibody according to any one of claims 1 to 7, for use as a medicament.

13. The antagonist antibody according to any one of claims 1 to 7, for its use for treating a disease or disorder selected in the group consisting of proliferative diseases, bacterial or viral infections including sepsis.
